(19) Europäisches Patentamt / European Patent Office / Office européen des brevets

(11) EP 2 655 636 B1

# (12) EUROPEAN PATENT SPECIFICATION

(45) Date of publication and mention of the grant of the patent: **22.04.2015 Bulletin 2015/17**

(21) Application number: **11850231.9**

(22) Date of filing: **22.12.2011**

(51) Int Cl.: ***C12N 15/67*** *(2006.01)* ***C12N 15/85*** *(2006.01)*

(86) International application number: **PCT/SG2011/000449**

(87) International publication number: **WO 2012/087246 (28.06.2012 Gazette 2012/26)**

## (54) MODIFIED CMV PROMOTERS THAT ARE RESISTANT TO GENE SILENCING

MODIFIZIERTE CMV-PROMOTOREN MIT RESISTENZ GEGEN GENAUSSCHALTUNG

PROMOTEURS DE CMV MODIFIÉ QUI SONT RÉSISTANTS AU SILENÇAGE DE GÈNES

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2010 US 201061427121 P**

(43) Date of publication of application: **30.10.2013 Bulletin 2013/44**

(73) Proprietor: **Agency for Science, Technology and Research Singapore 138632 (SG)**

(72) Inventors:
- **YANG, Yuansheng Singapore 138668 (SG)**
- **MARIATI, Mariati Singapore 138668 (SG)**

(74) Representative: **Viering, Jentschura & Partner Patent- und Rechtsanwälte Kennedydamm 55 / Roßstrasse 40476 Düsseldorf (DE)**

(56) References cited:
**WO-A2-2004/067701 WO-A2-2009/146668**

- **WILLIAMS S. ET AL.: "CpG-island fragments from the HNRPA2B1/CBX3 genomic locus reduce silencing and enhance transgene expression from the hCMV promoter/enhancer in mammalian cells", BMC BIOTECHNOLOGY, vol. 5, 17, 3 June 2005 (2005-06-03), pages 1-9, XP002722927,**
- **SENIGL, F. ET AL.: 'The core element of a CpG island protects avian sarcoma and leukosis virus-derived vectors from transcriptional silencing' JOURNAL OF VIROLOGY vol. 82, no. 16, 2008, pages 7818 - 7827, XP002556302**
- **HEJNAR, J. ET AL.: 'CpG island protects Rous sarcoma virus-derived vectors integrated into nonpermissive cells from DNA methylation and transcriptional suppression' PROCEEDINGS OF THE NATIONAL ACADEMY OF SCIENCES OF THE UNITED STATES OF AMERICA vol. 98, no. 2, 2001, pages 565 - 569, XP002556300**
- **KESHET, I. ET AL.: 'Effect of regional DNA methylation on gene expression' PROCEEDINGS OF THE NATIONAL ACADEMY OF SCIENCES OF THE UNITED STATES OF AMERICA vol. 82, 1985, pages 2560 - 2564, XP055112528**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention lies in the field of molecular biology, virology and gene therapy, and particularly relates to a nucleic acid molecule as recited in the claims containing a functional promoter of a herpesvirus, a functional enhancer of a herpesvirus and one or more internal elements of the CpG island of the aprt (adenine phosphoribosyl transferase) gene, including uses as recited in the claims of the nucleic acid molecule and methods as recited in the claims of producing a polypeptide or protein of interest.

### BACKGROUND OF THE INVENTION

The majority of studies in gene therapy research to date have utilized viral promoters. In general, strong viral promoters are required for efficient viral propagation, and they frequently induce much higher levels of transcription than eukaryotic promoters by using mechanisms to control and recruit host transcription machinery. Moreover, viral promoters tend to be far more compact and hence easier to manipulate and accommodate into gene therapy vectors.

Human cytomegalovirus major immediate early gene promoter (hCMV) has been widely used in both academic research and industrial applications for high level recombinant protein production in mammalian cells. For instance, the enhancer/promoter element driving immediate/early gene expression in hCMV has been widely used in biotechnology for driving expression of heterologous genes in eukaryotic expression vectors. Other viral promoters in use include the simian virus 40 (SV40), Rous sarcoma virus long terminal repeat (RSV-LTR), Moloney murine leukaemia virus (MoMLV) LTR, and other retroviral LTR promoters. The use of viral promoters has thus been widespread and successful *in vitro* and for certain applications *in vivo*. However, eukaryotic cells have evolved mechanisms to detect and silence viral transgene expression. Viral promoters have manifested a frequent inability to sustain transgene expression *in vivo*, but despite considerable evidence that viral promoters are prone to inactivation and silencing *in vitro* and *in vivo*, a large proportion of gene therapy applications continue to utilize them to drive transgene expression. Nonetheless, it is a general problem in that heterologous genes upon transduction into mammalian cells are expressed to a sufficient level transiently which is then suppressed and gradually silenced in stably transfected cell lines during long term culture. Gene silencing is therefore a major obstacle in gene therapy.

The loss in transgene expression is largely attributable to transcriptional silencing which involves methylation at CpG DNA sequences, histone deacetylation or chromatin condensation in the vicinity of the integration site. CpG islands are usually at least 200 bp long, GC rich, and contain at least about 60% frequency of CpG dinucleotides. DNA methylation is a covalent modification in which the 5' position of cytosine is methylated. In mammals, this modification occurs at CpG dinucleotides. In open chromatin where histones are widely dispersed, DNA is unmethylated, represented by open circles (Figure 1), gene is actively expressed. Upon methylation, methyl binding (MBD) proteins recruit histone modification proteins and co-repressors to the methylated region, causing chromatin to become condensed, and the gene is silenced.

Various anti-methylation techniques have been applied to prevent gene silencing. For example, Senigl, H and colleagues (Senigl, H; Plachy, J.; Hejnar, J., The core element of a CpG island protects avian sarcoma and leukosis virus-derived vectors from transcriptional silencing (2008) Journal of Virology, 82: 7818-7827) showed that insertion of the internal element (IE) of a CpG island between the enhancer and promoter of a small sized viral promoter, RSV LTR (~200 bases) prevented gene silencing. However, this strategy may not work for larger size promoters since it has been reported that IE can protect only about 150 bases from methylation (Siegfried, Z.; Eden, S., etc. DNA methylation represses transcription in vivo (1998) Nature genetics 22: 203-206).

UCOE (Ubiquitous Chromatin Opening Element) has been used in research and biotechnological applications such as recombinant protein products in cell culture. UCOE confer an increased proportion of productive gene integration events with improvements in the level and stability of transgene expression. Although UCOE was referred in the art as CpG island related elements (WO 2006/095156; Williams S. et al (2005), BMC Biotechnology, 5:17), UCOE is known to have a different function that is to structurally loosen chromatin and thus enable expression even if genes are integrated into heterochromatin. It is thus not clear whether UCOE can prevent DNA methylation of the respective nucleic acid.

Generation of cell lines that can maintain high productivity during the extended scale-up period is of the utmost importance for biopharmaceutical manufacturing. Production instability can affect product yield and product consistency, compromising regulatory approval of the product. Most clones generated using currently available expression vectors have unstable productivity. As a result, a large number of clones need to be screened to obtain stable high-producing cell lines. It has been indicated in previous literature reports that the loss in productivity was mainly due to transcriptional silencing which involved methylation of CpG dinucleotides within promoters.

Given the importance of recombinant protein expression in biotechnology, there remains a need for improved expression vectors comprising improved promoter or enhancer combinations.

## SUMMARY OF THE INVENTION

The invention in its broadest sense is as defined in the independent claims. In one aspect, the invention provides a nucleic acid molecule comprising a functional promoter of a cytomegalovirus, a functional enhancer of a cytomegalovirus, and one or more internal elements of the CpG island of the aprt (adenine phosphoribosyl transferase) gene, wherein the at least one of the one or more internal elements of the CpG island of the aprt gene comprises one or more binding sites for the transcription factor Sp1, wherein (1) the aprt gene is a hamster aprt gene and the Sp1 binding site of the CpG island of the aprt gene has the sequence: 5'-GCCCCGCCCCGTCCCGCCCC-3'(SEQ ID NO: 1); or (2) the aprt gene is the mouse aprt gene and the Sp1 binding site of the CpG island of the aprt gene has the sequence 5'-CCCGCCC-3' (SEQ ID NO: 2) or the sequence 5'-TCCGCCC-3' (SEQ ID NO: 3).

In a second aspect, the invention provides a method of producing a desired polypeptide in cell culture. The method includes providing a nucleic acid molecule as defined herein, wherein the nucleic acid molecule further comprises a nucleotide sequence encoding the desired polypeptide, the nucleotide sequence being operably linked to the promoter of the cytomegalovirus and the enhancer of the cytomegalovirus, and allowing expression of the desired polypeptide.

In a third aspect, the invention provides a vector comprising the nucleic acid molecule as defined herein.

In a fourth aspect, the invention provides a host cell comprising the nucleic acid molecule as defined herein.

In a fifth aspect, the invention provides use of the nucleic acid molecule as defined herein for enhancing the expression of a polypeptide of interest in cell culture, wherein the nucleic acid molecule comprises a nucleotide sequence coding for the polypeptide of interest, the nucleotide sequence being operably linked to the promoter of the cytomegalovirus and the enhancer of the cytomegalovirus.

## BRIEF DESCRIPTION OF THE DRAWINGS

The invention will be better understood with reference to the detailed description when considered in conjunction with the non-limiting examples and the accompanying drawings, in which:

**Figure 1** illustrates a schematic representation of the mechanism behind transcriptional gene silencing. Figure 1A shows the covalent modification of a nucleotide in which the 5' position of cytosine is methylated. Figure 1B shows the DNA methylation occurring at CpG dinucleotides. In open chromatin where histones ("H3") are widely dispersed, DNA is unmethylated, represented by open circles, gene is actively expressed. Upon methylation, methyl binding ("MBD") proteins recruit histone modification proteins (such as "HDAC": histone deacetylases) and co-repressors (such as "HP1": heterochromatin protein 1) to the methylated region, causing chromatin to become condensed, and gene is silenced.

**Figure 2** shows the nucleotide sequence (SEQ ID NO: 4) of the internal element (IE) of the CpG island found on hamster APRT gene. The internal element (IE) is a small region of CpG island of hamster APRT gene (Brandeis, M.; Frank, D.; Keshet, I., etc. Sp1 Elements Protect A Cpg Island From De-Novo Methylation (1994) Nature 371: 435-438).

**Figure 3A** shows the nucleotide sequence (SEQ ID NO: 6) containing human cytomegalovirus (CMV) enhancer and promoter. The CG dinucleotides are indicated in bold, which all could be methylated resulting in gene silencing. The CTCGAG sequence that is underlined and bold indicates the XhoI site generated using site-directed mutagenesis between the enhancer and promoter for insertion of IE.

**Figure 3B** shows a schematic representation of the nucleotide sequence of Figure 3A. Each circle refers to a CG dinucleotide in the sequence. The CG dinucleotides all could be methylated resulting in gene silencing.

**Figure 4** shows a schematic representation of twelve different nucleic acid molecules according to various embodiments of the invention, in which the one or two IEs of the CpG island of the aprt gene is/are inserted upstream of enhancer, between the enhancer and promoter (P), and downstream of promoter (P) in forward or reverse orientations, in order to study the location, orientation, and copy number effects on IE's ability of preventing gene silencing and whether insertion of IE affect promoter strength. The wild type CMV is referred as WT CMV and modified CMV is referred as IE CMV later on.

**Figure 5** shows the vector sequences containing the CMV enhancer and promoter (P) regions for expression of cloned DNA inserts in mammalian cells. MluI is for insertion of IE upstream of CMV enhancer, XhoI is for insertion of IE between CMV enhancer and promoter, NheI is for insertion of IE downstream of promoter. IRESatt, attenuated internal ribosome entry site (IRES). IRES allows expression of multiple genes under the control of one promoter. Attenuation of IRES's translation efficiency is for more efficient selection of high producing clones. mNPT, mutated neomycin with weakened enzyme activity which allows more efficient selection of high producing clones.

Figure 6 shows a schematic process of clone generation and evaluating the long term expression of the nucleic acid molecules of the invention in cell culture.

**Figure 7** shows the comparison of strength of the twelve nucleic acid molecules containing the modified CMV promoter ("IE CMV", see Figure 4) with the wild type CMV promoter ("WT CMV") in transient (Figure 7A) and stable gene expression (Figure 7B). The objective of insertion of IE into CMV was to enhance expression stability but without compromising expression level. The strength of the modified CMV promoters in expressing a gene was compared with the WT CMV in transient and stable transfections using GFP as a reporter gene. The mean fluorescence intensity of transiently transfected pool and stable expressing clones was measured using FACS. Significant differences relative to the WT CMV at a 95% confidence level are denoted by asterisks. Without wishing to be bound by theory, insertion of IE downstream of CMV decreased expression level in transient transfection, while insertion into other locations appear to have minor effect. In stable transfections, UF2, UR2, and MR1 had comparable expression level with wild type CMV.

**Figure 8** shows the percentage of GFP positive cells at week 8 and 0 of a representative clone B4 generated using WT CMV. At week 0, all cells expressed GFP. After 8 weeks passaging without selection reagent G418, only 34% cells still expressed GFP.

**Figure 9** shows the percentage of GFP positive cells at week 0 and 8 of a representative clone B8 using a IE CMV promoter (MF2) according to various embodiments of the invention. The IE CMV promoter (MF2) contains 2 IEs inserted between the enhancer and mini-promoter (Figure 9A). At week 0, all cells expressed GFP. After 8 weeks passaging without selection reagent G418, all cells still expressed GFP.

**Figure 10** shows the histograms of a representative clone B4 generated using WT CMV at week 0 and week 8. The retention of GFP expression (%) is calculated as the ratio of mean fluorescence intensity (MFI) at week 8 over that at week 0 as follows:

$$\text{Retention of GFP expression\%} = \frac{\text{MFI (week 8)}}{\text{MFI (week 0)}}$$

**Figure 11** shows the comparison of maintaining GFP expression using the nucleic acid molecules containing the modified CMV promoters (IE CMV, see Figure 4) and the wild type CMV (WT CMV) during 8 week-passage in CHO cells using GFP as reporter gene. 9 clones were isolated from 3 stable pools for each vector. They were cultured over 8 weeks without the presence of selection pressure. The GFP expression of each clone generated using the WT and IE CMV promoters was monitored over 8 weeks by using FACS as a measure of production stability. Figure 11A refers to the percentage of GFP positive cells. Figure 11B refers to percentage retention of GFP expression. Before stability testing, all clones had 100% of GFP positive cells. After 8-week passaging, GFP positive cells in most wild type clones dropped below 100%, and on average, less than 20% of their original expression levels were maintained after 8 weeks passaging. On the contrary, most clones generated using the IE CMV, such as UF2, UR2, MF2, MR1, MR2, and DR2 still had close to 100% GFP positive cells, and on average, close to or over 40% of their original expression levels were maintained in clones generated using UF1, UR2, MR1, MR2, DF2, and DR2.

**Figure 12** shows the schematic representation of three nucleic acid molecules containing the modified hCMV promoters (IE CMV) according to various embodiments of the invention namely, "UR2" wherein 2 IEs were inserted upstream of the enhancer in reverse orientation; "MR1" wherein 1 IE was inserted between the enhancer and promoter in reverse orientation; and "UR2 + MR1" containing 2 IEs (reverse orientations) inserted upstream of enhancer and 1 IE (reverse orientation) inserted between the enhancer and promoter. 18 clones were isolated from 3 stably transfected pool for each vector for comparison of strength and maintenance of expression level during long term culture.

**Figure 13** shows the comparison of the strength of the modified CMVs (IE CMVs) namely MR1, UR2 and UR2+MR1 (see Figure 12) with the wild type (WT CMV) in stable expression. Consistent with results in Figure 11, UR2 and MR1 showed comparable strength to WT CMV in expressing a gene. However, combination of UR2+MR1 resulted in lower gene expression levels.

**Figure 14** shows the comparison of the modified CMVs namely MR1, UR2 and UR2+MR1 with the wild type (WT CMV) for maintaining proportion of GFP positive cells after 8 week-passage. Consistent with results in Figure 11, UR2 and MR1 enhanced proportion of GFP positive cells compared to WT CMV during 8 week-passaging. However, combination of UR2+MR1 did not further enhance expression stability.

**Figure 15** shows the comparison of the modified CMVs wamely MR1, UR2 and UR2+MR1 with the wild type (WT CMV) for retention of expression after 8 week-passage. Consistent with results in Figure 11, UR2 and MR1 enhanced expression stability compared to WT CMV during 8 week-passaging. However, combination of UR2+MR1 did not further enhance expression stability.

**Figure 16** shows a schematic representation of two vector sequences as follows: A) "wild type mCE' containing mouse CMV enhancer and human EF1$\alpha$ promoter (mcE ordered from Invivogen); and B) "mCE + 2IE" in which 2 IEs were applied on the promoter consisting of the mouse CMV enhancer and human EF1$\alpha$ promoter. 9 clones

were isolated from 3 stably transfected pool for each vector for comparison of strength and maintenance of expression level during long term culture.

**Figure 17** shows the comparison of strength of the modified mCE (mCE + 2IE) with wild type mCE in stable expression. Insertion of 2IE into mCE resulted in lower expression level as compared to the wild type mCE.

**Figure 18** shows the comparison of the modified mCE (mCE + 2IE) with wild type mCE for maintaining proportion of GFP positive cells after 8 week-passage. Insertion of 2IE into mCE resulted in lower proportion of GFP positive cells as compared to the wild type mCE.

**Figure 19** shows the comparison of the modified mCE (mCE + 2IE) with wild type mCE for retention of expression after 8 week-passage. It appears that the insertion of 2 IE between the enhancer and promoter did not improve GFP expression stability. These results, together with those in Figure 18, indicated that the effect of the IE is promoter specific.

**Figure 20** illustrates the anti-silencing mechanisms of the nucleic acid molecule MR1 containing 1 IE in the reverse orientation inserted between the enhancer and mini-promoter (see Figure 4). Three clones generated using the WT CMV and MR1 respectively were characterized for changes in GFP expression level, gene copy number, and mRNA level during 8 weeks passaging. The GFP gene copy number and mRNA level were determined using qRT-PCR and normalized to β-actin. Figure 20A shows the changes in GFP expression levels, Figure 20B shows the changes in GFP gene copy numbers and Figure 20C shows the changes in GFP mRNA levels between WT CMV and MR1. GFP gene copy numbers in clones generated using WT CMV remained unchanged during long term culture while mRNA levels dropped and correlated to the decrease in mean fluorescence intensity, suggesting the loss in GFP expression level was due to transcriptional silencing. In contrast, clones generated using MR1 maintained GFP gene copy number and mRNA levels during long term culture, thus exhibited less drop in expression levels.

## DETAILED DESCRIPTION OF THE INVENTION

The invention in its broadest sense is as defined in the independent claims.

The invention is based on the surprising finding that the insertion of one or more core CpG island elements (IE) into the promoter region of a cytomegalovirus at specified locations and orientations can prevent a nucleic acid molecule as recited in the claims, in particular, DNA, from methylation, so that gene expression levels can be maintained but without compromising the promoter strength. In particular, the modified promoter region of the cytomegalovirus is more resistant to gene silencing due to the presence of one or more IEs. Therefore, this strategy can be applicable for protection of large sized promoters. However, it was also found that not all so-called CpG island related elements are able to perform the function of preventing gene silencing in any nucleic acid sequence (see for example, Figures 17-19), but that this functionality may be promoter-specific. It was thus a surprising finding that an internal element of the aprt (adenine phosphoribosyl transferase) gene can prevent gene silencing within the nucleic acid sequence of a cytomegalovirus. In this context, the SP1 binding sites within the internal element of the mouse aprt gene were found to be critical for demethylation.

The present invention provides a nucleic acid molecule comprising a functional promoter of a cytomegalovirus, a functional enhancer of a cytomegalovirus, and one or more internal elements of the CpG island of the aprt (adenine phosphoribosyl transferase) gene, wherein the at least one of the one or more internal elements of the CpG island of the aprt gene comprises one or more binding sites for the transcription factor Sp1, wherein (1) the aprt gene is a hamster aprt gene and the Sp1 binding site of the CpG island of the aprt gene has the sequence: 5'-GCCCCGCCCCGTCCCGCCCC-3'(SEQ ID NO: 1); or (2) the aprt gene is the mouse aprt gene and the Sp1 binding site of the CpG island of the aprt gene has the sequence 5'-CCCGCCC-3' (SEQ ID NO: 2) or the sequence 5'-TCCGCCC-3' (SEQ ID NO: 3).

The aprt gene is selected from the group consisting of the hamster aprt gene and the mouse aprt gene. When the aprt gene of a hamster is desired, the hamster can be of any genus for example *Mesocricetus, Phodopus, Cricetus, Cricetulus, Allocricetulus, Cansumys* and *Tscherskia.* Any subspecies of a hamster can be used and may include *Cricetulus griseus, Cricetulus sp., Cricetulus alticola, Cricetulus barabensis, Cricetulus kamensis, Cricetulus longicaudatus, Cricetulus migratorius* and *Cricetulus sokolovi.* When the aprt gene of a mouse is desired, the mouse can be of any subspecies for example *Mus musculus, Peromyscus leucopus,* or *Peromyscus maniculatus.*

An "internal element", as used in the context of the present invention, refers to a nucleotide stretch that prevents gene silencing by preventing DNA methylation of itself and the promoter region, which includes the enhancer and the minimal promoter.

In various embodiments, the one or more internal elements of the CpG island of the aprt gene includes one or more binding sites for the transcription factor Sp1. When the aprt gene is the hamster gene, the SP1 binding site of the CpG island of the aprt gene has the sequence: 5'-GCCCCGCCCCGTCCCGCCCC-3' (SEQ ID NO: 1). When the aprt gene is the mouse aprt gene, the SP1 binding site of the CpG island of the aprt gene has the sequence 5'-CCCGCCC-3' (SEQ ID NO: 2) or the sequence 5'-TCCGCCC-3' (SEQ ID NO: 3). When the aprt gene is the hamster aprt gene, the internal element of the CpG island of the aprt gene has the sequence:

5'-
TCCAGCAAATGCGTTACTTCCTGCCAAAAGCCAGCCTCCCCGCAACCCACT
CTCCCAGAGGCCCCGCCCCGTCCCGCCCCCTCCCGGCCTCTCCTCGTGCTG
GATCGCTCCCTAAGGA-3' (SEQ ID NO: 4).

When the aprt gene is the mouse aprt gene, the internal element of the CpG island of the aprt gene has the sequence:

5'-
AGGATGGACATCGCACATCCCCTTTCCACCCATATATCTTTGAGGTAGGGA
TGCTTGTGTTTAGGCAGCTCAAGAAATCTAACCCCTGACTCAGGCCCCACA
CACACCTCGCAGAGGCCCCGCCTCTCAGCCTGTCCCGCCCCTCGTGCTAGA
CCAACCCGCACCCAGAAGCCCCGCCCATCGAGGACGCTCCGCCCTTGTTC
CCCCCGGGATTGACGTG-3' (SEQ ID NO: 5).

In various embodiments, one or more internal elements of the CpG island of the aprt gene can be independently arranged in the nucleic acid molecule of the invention in the forward orientation (sense) or in the reverse orientation (antisense), relative to the sequence of the promoter. A plurality of internal elements for example two, three, four, five or more internal elements of the CpG island of the aprt gene and/or of the functional variant thereof can be independently arranged in the nucleic acid molecule of the invention.

In various embodiments, the nucleic acid molecule can further include an expressible nucleotide sequence coding for a polypeptide of interest. The expressible nucleotide sequence is operably linked to the promoter of the cytomegalovirus and the enhancer of the cytomegalovirus. An operable linkage is a linkage in which the regulatory DNA sequences according to the invention and the DNA sequences sought to be expressed are connected in such a way as to permit gene sequence expression. Therefore, the expressible nucleotide sequence can be transcribed from the functional promoter of the cytomegalovirus. The level of transcription is enhanced and/or stabilized over periods of about 10, about 20, about 30, about 40, about 50, about 60 or more cycles of expression by the internal element of the CpG island of an aprt gene.

In various embodiments, the expressible nucleotide sequence is a heterologous nucleotide sequence. The expressible nucleotide sequence can in some embodiments be arranged downstream from the promoter of the cytomegalovirus and the enhancer of the cytomegalovirus. The term "heterologous" when used in reference to a nucleotide sequence or nucleic acid molecule, means a nucleotide sequence not naturally occurring in the respective cell into which the nucleic acid molecule has been (or is being) introduced. A heterologous nucleic acid sequence thus originates from a source other than the respective cell and can occur naturally or non-naturally. A respective heterologous nucleic acid sequence may for example be integrated into the nucleic acid molecule of the present invention.

The functional promoter and the enhancer can be of the same cytomegalovirus or of different types of cytomegalovirus. In various embodiments, cytomegalovirus is a human cytomegalovirus (hCMV).

The nucleic acid molecule of the cytomegalovirus contains a promoter region (which includes the functional enhancer and the minimal promoter) and can be of any length. In some embodiments, the promoter regions can be of a size of at least about 300 base pairs, a size of about 350 base pairs or more, about 400 base pairs or more, about 450 base pairs or more, about 500 base pairs or more, about 550 base pairs or more or about 600 base pairs or more.

In various embodiments, the functional promoter and the functional enhancer can be comprised in the sequence:

5'-TTGACATTGATTATTGACTAGTTATTAATAGTAATCAATTACGGGGTCATTAGTTCATAGCCCATATATGGAGTTCCGCGTTACATAACTTACGGTAAATGGCCCGCCTGGCTGACCGCCCAACGACCCCCGCCCATTGACGTCAATAATGACGTATGTTCCCATAGTAACGCCAATAGGGACTTTCCATTGACGTCAATGGGTGGAGTATTTACGGTAAACTGCCCACTTGGCAGTACATCAAGTGTATCATATGCCAAGTACGCCCCCTATTGACGTCAATGACGGTAAATGGCCCGCCTGGCATTATGCCCAGTACATGACCTTATGGGACTTTCCTACTTGGCAGTACATCTACGTATTAGTCATCGCTATTACTCGAGTGATGCGGTTTTGGCAGTACATCAATGGGCGTGGATAGCGGTTTGACTCACGGGGATTTCCAAGTCTCCACCCCATTGACGTCAATGGGAGTTTGTTTTGGCACCAAAATCAACGGGACTTTCCAAAATGTCGTAACAACTCCGCCCCATTGACGCAAATGGGCGGTAGGCGTGTACGGTGGGAGGTCTATATAAGCAGAGCTC-3' (SEQ ID NO: 6).

In various embodiments, the functional promoter has the sequence:

5'-TGATGCGGTTTTGGCAGTACATCAATGGGCGTGGATAGCGGTTTGACTCACGGGGATTTCCAAGTCTCCACCCCATTGACGTCAATGGGAGTTTGTTTTGGCACCAAAATCAACGGGACTTTCCAAAATGTCGTAACAACTCCGCCCCATTGACGCAAATGGGCGGTAGGCGTGTACGGTGGGAGGTCTATATAAGCAGAGCTC-3' (SEQ ID NO: 7).

In various embodiments, the functional enhancer has the sequence:

5'-TGACATTGATTATTGACTAGTTATTAATAGTAATCAATTACGGGGTCATTAGTTCATAGCCCATATATGGAGTTCCGCGTTACATAACTTACGGTAAATGGCCCGCCTGGCTGACCGCCCAACGACCCCCGCCCATTGACGTCAATAATGACGTATGTTCCCATAGTAACGCCAATAGGGACTTTCCATTGACGTCAATGGGTGGAGTATTTACGGTAAACTGCCCACTTGGCAGTACATCAAGTGTATCATATGCCAAGTACGCCCCCTATTGACGTCAATGACGGTAAATGGCCCGCCTGGCATTATGCCCAGTACATGACCTTATGGGACTTTCCTACTTGGCAGTACATCTACGTATTAGTCATCGCTATTA-3' (SEQ ID NO: 8).

Typically, the enhancer of the cytomegalovirus is arranged upstream of the promoter of the cytomegalovirus.

In various embodiments, at least one of the one or more internal elements of the CpG island of the aprt gene, or the functional variant thereof, is arranged upstream of the promoter of the cytomegalovirus or adjacently downstream of the promoter of the cytomegalovirus. When the one or more internal elements of the CpG island of the aprt gene, or the functional variant thereof is arranged upstream of the promoter of the cytomegalovirus, the said internal element(s) can be arranged (i) between the enhancer of the cytomegalovirus and the promoter of the cytomegalovirus or (ii) adjacently upstream of the enhancer of the cytomegalovirus.

In various embodiments, at least one of the one or more internal elements of the CpG island of the aprt gene, or the functional variant thereof, is arranged adjacent to the enhancer of the cytomegalovirus or adjacent to the promoter of the cytomegalovirus. "adjacent" or "adjacently" as used herein in relation to nucleic acid sequence elements includes embodiments wherein the 3' end of one sequence element directly connects to the 5' of the other sequence element. In other embodiments, the respective sequence elements may be separated by less than 20 additional nucleotides that belong to neither the first nor the second sequence element, but rather define a linker-type sequence. If the two sequences are separated by such a linker-like sequence, they may still be in frame. In such embodiments, the linker-like additional nucleotide sequence may be 3, 6, 9, 12, 15, or 18 nucleotides long.

The one or more internal elements of the CpG island of the aprt gene can be independently arranged either in the forward (sense) or reverse (antisense) orientation in the nucleic acid molecule of the invention. In specific embodiments, the nucleic acid molecules of the invention can be illustrated in Figure 4, wherein 1) one or more internal elements of the CpG island of the aprt gene is arranged adjacently upstream of the enhancer of the cytomegalovirus in the forward or reverse orientations (see UF1, UF2, UR1 and UR2); 2) one or more internal elements of the CpG island of the aprt gene is arranged between the enhancer and the promoter of the cytomegalovirus in the forward or reverse orientations (see MF1, MF2, MR1 and MR2); and 3) one or more internal elements of the CpG island of the aprt gene is arranged adjacently downstream of the promoter of the cytomegalovirus in the forward or reverse orientations (see DF1, DF2, DR1 and DR2).

As used herein, "nucleic acid" and "nucleic acid molecule" are used interchangeably and refer to any acid in any possible configuration, such as linearized single stranded, double stranded or a combination thereof. Nucleic acids may include, but are not limited to DNA molecules (e.g., cDNA or genomic DNA), RNA molecules (e.g., mRNA), analogues of the DNA or RNA generated using nucleotide analogues or using nucleic acid chemistry, cDNA synthetic DNA, a copolymer of DNA and RNA, oligonucleotides, and PNA (protein nucleic acids). DNA or RNA may be of genomic or synthetic origin and may be single or double stranded. A respective nucleic acid may furthermore contain non-natural nucleotide analogues and/or be linked to an affinity tag or a label. Also encompassed are nucleic acid analogues, such as peptide nucleic acids and the like.

As used herein, the general term "nucleotides" include nucleoside mono-, di-, and triphosphates. However, in the context of a nucleotide sequence, i.e. an oligo- or polynucleotide, the nucleotides are polymerized with a phosphate backbone, i.e. are monophosphates. The term "nucleotides" also includes modified nucleotides, such as, but not limited to, phophorothioate nucleotides and deazapurine nucleotides, 2'-methoxy nucleotides and other nucleotide analogs.

In various embodiments, the nucleic acid molecule according to the invention is a DNA molecule. In some embodiments, the functional promoter of the cytomegalovirus is the only promoter comprised in the nucleic acid molecule.

In various embodiments, the nucleic acid molecule of the invention is comprised in a vector. The term "vector" relates to a single or double-stranded circular nucleic acid molecule that can be introduced, e.g. transfected, into cells and replicated within or independently of a cell genome. A circular double-stranded nucleic acid molecule can be cut and thereby linearized upon treatment with restriction enzymes. An assortment of nucleic acid vectors, restriction enzymes, and the knowledge of the nucleotide sequences cut by restriction enzymes are readily available to those skilled in the art. A nucleic acid molecule according to the present invention can be inserted into a vector by cutting the vector with restriction enzymes and ligating the two pieces together. Therefore, the invention also provides a vector comprising a nucleic acid molecule of the invention. In some embodiments, the vector is an eukaryotic or a prokaryotic expression vector. In other embodiments, the vector is a plasmid. Commercially available plasmids such as pBR322, puC19, pBluescript and the like may be used. Typically, mammalian expression vectors can be used and are commercially available. Examples of mammalian expression vectors can include but are not limited to pCDM8 (Seed, B. Nature, 1987, 329: 840), pMT2PC (Kaufinan et al, 1987, EMBO J. 6:187-195), pCI and pSI mammalian vectors.

In some embodiments, the nucleic acid molecule is comprised in a suitable host cell. The host cell comprising the nucleic acid molecule of the invention can be an eukaryotic or a prokaryotic cell. In this context, the (transformed) host cells can be cultured under conditions suitable for expression of the nucleic acid molecule of the invention. Host cells can be established, adapted and completely cultivated under serum free conditions, and optionally in media which are free of any protein/peptide of animal origin. Commercially available media such as RPMI-1640 (Sigma), Dulbecco's Modified Eagle's Medium (DMEM; Sigma), Minimal Essential Medium (MEM; Sigma), CHO-S-SFMII (Invitrogen), serum free-CHO Medium (Sigma), and protein-free CHO Medium (Sigma) are exemplary appropriate nutrient solutions. Any of the media may be supplemented as necessary with a variety of compounds, examples of which are hormones and/or other growth factors (such as insulin, transferrin, epidermal growth factor, insulin like growth factor), salts (such as sodium chloride, calcium, magnesium, phosphate), buffers (such as HEPES), nucleotides (such as adenosine, thymidine), glutamine, glucose or other equivalent energy sources, antibiotics, trace elements. Any other necessary supplements may also be included at appropriate concentrations that are known to those skilled in the art.

In some embodiments, the nucleic acid molecule is an isolated nucleic acid molecule. An "isolated" nucleic molecule of the present invention can refer to one that is separated from other nucleic acid molecules which are present in the natural source of the nucleic acid molecule. An isolated molecule, for example a DNA molecule, can be substantially free of other cellular material, or culture medium when produced by recombinant techniques, or substantially free of chemical precursors or other chemicals when chemically synthesized. In other words, an isolated nucleic acid molecule can be free or substantially free of sequences (for example protein-encoding sequences) which flank the nucleic acid (i.e., sequences located at the 5' and 3's ends of the nucleic acid) in the genomic DNA of a cell or organism from which the nucleic acid is derived.

Typically, the term "functional variant", as used herein, refers to a sequence with at least 75%, at least 80%, at least 85%, at least 90%, at least 95%, at least 97%, or at least 99% sequence, or 75%, 80%, 85%, 90%, 91%, 92%, 93%, 94%, 95%, 96%, 97%, 98%, 99% identity to its respective original sequence, i.e. usually the corresponding aprt gene. By "sequence identity" is meant a property of sequences that measures their similarity or relationship. This term refers to the percentage of pair-wise identical residues obtained after a homology alignment of a nucleic acid sequence, of an aprt gene with a nucleic acid sequence, respectively, in question, wherein the percentage figure refers to the number of residues in the longer of the two sequences.

Also encompassed by the present invention are nucleic acid sequences substantially complementary to the above nucleic acid sequence. "Substantially complementary" as used herein refers to the fact that a given nucleic acid sequence is at least 90, for instance at least 95, and in some embodiments 100 % complementary to another nucleic acid sequence. The term "complementary" or "complement" refers to two nucleotides that can form multiple favourable interactions with one another. Such favourable interactions include and may exclusively be Watson-Crick base pairing. A nucleotide sequence is the full complement of another nucleotide sequence if all of the nucleotides of the first sequence are complementary to all of the nucleotides of the second sequence.

The invention also relates to a method of producing a desired polypeptide in cell culture. The method comprising: providing a nucleic acid molecule according to the present invention, wherein the nucleic acid molecule further comprises a nucleotide sequence coding for the desired polypeptide, the nucleotide sequence being operably linked to the promoter of the cytomegalovirus and the enhancer of the cytomegalovirus, and allowing expression of the desired polypeptide.

The term "polypeptide" as used herein is intended to cover naturally occurring proteins, as well as those which are recombinantly or synthetically produced. The term "polypeptide" is not limited to a specific length of the encoded product and, therefore, encompasses peptides, oligopeptides, and proteins. The term "polypeptide" also encompasses two or more polypeptides combined to form the encoded product. Polypeptides also include hybrid polypeptides which comprise a combination of partial or complete polypeptide sequences obtained from at least two different polypeptides wherein one or more may be heterologous to the cell in which the polypeptide is expressed. Polypeptides further include naturally occurring allelic and engineered variations of the above mentioned polypeptides and hybrid polypeptides.

In some embodiments, the nucleotide sequence encoding the desired polypeptide is a heterologous nucleotide sequence.

In some embodiments, the step of allowing expression of the desired polypeptide comprises introducing the nucleic acid molecule according to the present invention into a suitable host cell. In some embodiments, the nucleic acid molecule according to the present invention is comprised in a vector suitable for expression in the host cell.

The host cell may be any suitable cell, such as a eukaryotic cell. The eukaryotic cell can, for example be an animal cell, a plant cell (for e.g. monocots, dicots, algae), a fungus, a yeast cell, flagellum, microsporidia or protist. An animal cell can be derived from a mammal such as a primate, human, murine, bovine, rodent, human, insect, reptile, or a bird, to mention only a few. Examples of an animal cell such as a mammalian cell can include Chinese hamster ovary cells (for e.g. CHO-K1), COS cells (e.g., COS-1, COS-7), baby hamster kidney cells (BHK), human embryonic kidney (HEK) (e.g. HEK 293), Bowes melanoma cells, rat myeloma cells, mouse myeloma cells, antibody producing-hybridoma cells, human leukemia cells and the like. In some embodiments, the host cell is a neuron. In other embodiments, the host cell is a stem cell. A yeast cell can for example be *S. cerevisiae, S. pombe, C. albicans,* or *Saccharomycetale* cell.

In some embodiments, expression of the desired polypeptide is allowed in the host cell for over about 30 generations or more. Within the context of this embodiment, the level of expression of the desired polypeptide after about 30 generations or more of the host cell is enhanced when compared to a nucleic acid molecule with a heterologous nucleotide sequence coding for the polypeptide of interest that does not have an internal element of the CpG island of the aprt gene.

In some embodiments, the desired polypeptide is expressed in a plurality of host cells, and wherein the number of host cells still expressing the desired polypeptide after about 30 generations or more of the host cell may be enhanced when compared to a plurality of host cells comprising a nucleic acid molecule with a heterologous nucleotide sequence coding for the polypeptide of interest that does not have an internal element of the CpG island of the aprt gene.

In some embodiments, expression of the desired polypeptide is allowed over a period of about four weeks or more. Within the context of this embodiment, the level of expression of the desired polypeptide after the period of four weeks or more may be enhanced when compared to a nucleic acid molecule with a heterologous nucleotide sequence coding for the polypeptide of interest that does not have an internal element of the CpG island of the aprt gene.

In some embodiments, the desired polypeptide is expressed in a plurality of host cells, and wherein the number of host cells still expressing the desired polypeptide after the period of four weeks or more is enhanced when compared to a plurality of host cells comprising a nucleic acid molecule with a heterologous nucleotide sequence coding for the polypeptide of interest that does not have an internal element of the CpG island of the aprt gene.

In some embodiments, expression of the desired polypeptide is allowed in the host cell for over about 60 generations or more.

In some embodiments, expression of the desired polypeptide is allowed over a period of eight weeks or more. Within the context of this embodiment, the level of expression of the desired polypeptide after the period of eight weeks or more is enhanced when compared to a nucleic acid molecule that does not have an internal element of the CpG island of the aprt gene.

In some embodiments, the desired polypeptide is expressed in a plurality of host cells, and wherein the number of host cells still expressing the desired polypeptide after the period of eight weeks or more is enhanced when compared to a plurality of host cells comprising a nucleic acid molecule with a heterologous nucleotide sequence coding for the polypeptide of interest that does not have an internal element of the CpG island of the aprt gene.

The invention also provides a use of the nucleic acid molecule according to the present invention for enhancing the expression of a polypeptide of interest in cell culture, wherein the nucleic acid molecule comprises a nucleotide sequence coding for the polypeptide of interest, the nucleotide sequence being operably linked to the promoter of the cytomegalovirus and the enhancer of the cytomegalovirus.

In some embodiments, polypeptide of interest is expressed in a suitable host cell. In some embodiments expression of the polypeptide of interest is allowed in the host cell over about 30 generations or more. In some embodiments, expression of the polypeptide of interest is allowed in the host cell over about 60 generations or more.

In some embodiments, the level of expression of the desired polypeptide after about 30 generations or more of the host cell is enhanced when compared to a nucleic acid molecule with a heterologous nucleotide sequence coding for the polypeptide of interest that does not have an internal element of the CpG island of the aprt gene.

In some embodiments, the level of expression of the desired polypeptide after about 60 generations or more of the host cell is enhanced when compared to a nucleic acid molecule with a heterologous nucleotide sequence coding for the polypeptide of interest that does not have an internal element of the CpG island of the aprt gene.

In some embodiments, the desired polypeptide is expressed in a plurality of host cells, and wherein the number of host cells still expressing the desired polypeptide after about 30 generations or more of the host cell is enhanced when compared to a plurality of host cells comprising a nucleic acid molecule with a heterologous nucleotide sequence coding for the polypeptide of interest that does not have an internal element of the CpG island of the aprt gene.

In some embodiments, the desired polypeptide is expressed in a plurality of host cells, and wherein the number of host cells still expressing the desired polypeptide after about 60 generations or more of the host cell is enhanced when compared to a plurality of host cells comprising a nucleic acid molecule with a heterologous nucleotide sequence coding for the polypeptide of interest that does not have an internal element of the CpG island of the aprt gene.

In some embodiments, the internal element of the CpG island of the aprt gene prevents methylation of the functional promoter and/or the functional enhancer, thereby decreasing and/or preventing the reduction of the level of expression of the desired polypeptide.

The nucleic acid molecule of the present invention can for instance be used in recombinant protein production in cell culture, for example, in mammalian cells, cell engineering, protein engineering or gene therapy.

The following exemplary data illustrates the usefulness and advantages of a nucleic acid molecule according to the present invention.

## EXAMPLES

### Generation of IE CMV promoters

Generation of IE CMV and vectors for testing their ability to maintain gene expression during long term culture was summarized in Figure 4 and 5. The IE element was synthesized using PCR to anneal two primers, IE-Forward:

5'-TCCAGCAAATGCGTTACTTCCTGCCAAAAGCCAGCCTCCCCGCAACCCACTCTCCCAGAGGCCCCGCCCC-3' (SEQ ID NO: 12)

and IE-Reverse:

5'-TCCTTAGGGAGCGATCCAGCACGAGGAGAGGCCGGGAGGGGGCGGACGGGGCGGGGCCTCTGGGAGA-3' (SEQ ID NO: 13).

Two copies of IE were linked by overlapping PCR. The wild type CMV promoter in pCIN was cloned from pcDNA3.1(+) vector (Invitrogen, Carlsbad, CA). To generate IE CMV, a XhoI site was generated between CMV enhancer and minimal promoter (mP) using QuickChange site directed mutagenesis kit (Stratagene, La Jolla, CA). One copy or two copies of IE in either forward or reverse orientation were then inserted into upstream of CMV promoter using MluI site, between enhancer and mP using XhoI site, and downstream of CMV promoter using NheI site. All restriction enzymes and ligase used for vector construction were ordered from New England Biolabs (Ipswich, MA).

**FACS analysis of GFP expression in CHO cells**

The FACSCalibur™ system (Becton Dickinson, CA, USA) was used for estimating the green fluorescence levels of various clones. Green fluorescence at 525 nM was detected through FL1 set at a PMT voltage of 269 or 516, with a logarithmic gain. Ten thousand cells were analyzed for each sample. Data analysis was performed using Flowjo software.

**Analysis of GFP gene copy number and mRNA levels**

Genomic DNA and RNA were isolated from cells using PureGene DNA purification kit (Gentra Systems, Minneapolis, MN) and RNAqueous-4PCR kit (Ambion, Austin, TX), respectively. First-strand cDNA was prepared from mRNA using the ImProm-II Reverse Transcription System (Promega, Madison, WI). For each reverse transcription reaction 100 ng total RNA were used. The relative GFP transgene copy numbers and mRNA levels were determined using real-time quantitative PCR (qRT-PCR). β-actin, a housekeeping gene, served as the internal control to normalize for possible variation in input quantity of DNA or RNA between samples. qRT-PCR was performed by using the ABI Prism 7000 sequence Detection System in conjunction with the iTag SYBR Green Supermix (Bio-Rad, Hercules, CA). The collected data were analyzed using the $2^{-\Delta\Delta Ct}$ method (Livak and Schittgen, 2001, Methods 25 (4): 402-408).

**Example 1: Generation of single-cell clones**

The vectors containing different modified CMV promoters with IE inserted are transfected into CHO K1 cells using electroporation in 6-well plate. At 48 hr post-transfection, G418, the selection reagent, is added for selection of cells with vectors integrated into genome. After 2 to 3 weeks selection, most survived cells will stably express GFP. Single-cell clones are then obtained using limiting dilution method and banked.

**Example 2: Testing of long term gene expression**

Single-cell clones obtained in step 1 are thawed and grown in 6-well plate. At week 0, photos are taken for different clones under the microscopes to estimate percentage of GFP positive cells. The percentage of GFP positive cells for each clone and expression level are also quantitatively determined using FAC. The cells are then passaged in the absence of G418 for 8 weeks. Photos are taken and FACS analysis is done again and compared results at week 0.

A modified hCMV promoter which is more resistant to gene silencing by using a core CpG island element (IE) of the aprt gene is described herein. The wild type hCMV promoter consists of an enhancer and promoter. Without wishing to be bound by any theory, it is found that insertions of a single IE element upstream of the enhancer or between the enhancer and promoter improve the ability of hCMV to maintain long term gene expression and do not compromise promoter strength for high level expression. It is also found that insertion of IE downstream of the minimal promoter also protects the hCMV from gene silencing but inhibits gene expression. Therefore and without wishing to be bound by theory, the protective effect of IE at each location is different and dependent on its orientation.

Another advantage of using the nucleic acid molecules of the present invention is the generation of cell lines with high stability. All clones generated using this modified CMV contained 100% GFP positive cells and maintained GFP expression over 50% of the original level after 8 weeks passaging, as compared to clones generated using the wild type CMV which contained many non-expressing cells and maintained expression less than 20%. Also, this modified IE CMV has comparable strength with the wild type CMV in expressing a gene in both transient and stable transfections. This anti-silencing protection provided by the IE element should be beneficial for generation of cell lines with high stability.

The invention illustratively described herein may suitably be practiced in the absence of any element or elements, limitation or limitations, not specifically disclosed herein. Thus, for example, the terms "comprising", "including", "containing", etc. shall be read expansively and without limitation.

It should be understood that although the present invention has been specifically disclosed by a preferred embodiment, modification and variation of the invention herein disclosed may be resorted to by those skilled in the art, and that such modifications and variations are considered to be within the scope of this invention as recited in the claims.

## Claims

**1.** A nucleic acid molecule comprising a functional promoter of a cytomegalovirus, a functional enhancer of a cytomegalovirus, and one or more internal elements of the CpG island of the aprt (adenine phosphoribosyl transferase) gene, wherein at least one of the one or more internal elements of the CpG island of the aprt gene comprises one or more binding sites for the transcription factor Sp1, wherein

(1) the aprt gene is a hamster aprt gene and the SP1 binding site of the CpG island of the aprt gene has the sequence: 5'-GCCCCGCCCCGTCCCGCCCC-3'(SEQ ID NO: 1); or
(2) the aprt gene is the mouse aprt gene and the SP1 binding site of the CpG island of the aprt gene has the sequence 5'-CCCGCCC-3' (SEQ ID NO: 2) or the sequence 5'-TCCGCCC-3' (SEQ ID NO: 3).

**2.** The nucleic acid molecule of claim 1, wherein

(i) when the aprt gene is the hamster aprt gene, the internal element of the CpG island of the aprt gene has the sequence:

5'-
TCCAGCAAATGCGTTACTTCCTGCCAAAAGCCAGCCTCCCCGCAACCCAC
TCTCCCAGAGGCCCCGCCCCGTCCCGCCCCCTCCCGGCCTCTCCTCGTGCT
GGATCGCTCCCTAAGGA-3' (SEQ ID NO: 4);

or
(ii) when the aprt gene is the mouse aprt gene, the internal element of the CpG island of the aprt gene has the sequence:

5'-
AGGATGGACATCGCACATCCCCTTTCCACCCATATATCTTTGAGGTAGGG
ATGCTTGTGTTTAGGCAGCTCAAGAAATCTAACCCCTGACTCAGGCCCCA
CACACACCTCGCAGAGGCCCCGCCTCTCAGCCTGTCCCGCCCCTCGTGCTA
GACCAACCCGCACCCAGAAGCCCCGCCCATCGAGGACGCTCCGCCCTTGT
TCCCCCCGGGATTGACGTG-3' (SEQ ID NO: 5).

**3.** The nucleic acid molecule of claim 1 or 2, wherein the one or more internal elements of the CpG island of the aprt gene are independently arranged in the nucleic acid molecule in the forward orientation or in the reverse orientation, relative to the sequence of the promoter.

**4.** The nucleic acid molecule of any one of claims 1 to 3, wherein the nucleic acid molecule comprises a plurality of the internal element of the CpG island of the aprt gene.

**5.** The nucleic acid molecule of any one of claims 1 to 4, wherein the nucleic acid molecule further comprises an expressible nucleotide sequence coding for a polypeptide of interest, the expressible nucleotide sequence being operably linked to the promoter of the cytomegalovirus and the enhancer of the cytomegalovirus and preferably being a heterologous nucleotide sequence.

**6.** The nucleic acid molecule of claim 5, wherein the expressible nucleotide sequence is arranged downstream from the promoter of the cytomegalovirus and the enhancer of the cytomegalovirus.

**7.** The nucleic acid molecule of any one of claims 1 to 5, wherein

(1) the functional promoter and the functional enhancer are comprised in the sequence:

5'-
TTGACATTGATTATTGACTAGTTATTAATAGTAATCAATTACGGGGTCATT
AGTTCATAGCCCATATATGGAGTTCCGCGTTACATAACTTACGGTAAATG
GCCCGCCTGGCTGACCGCCCAACGACCCCCGCCCATTGACGTCAATAATG
ACGTATGTTCCCATAGTAACGCCAATAGGGACTTTCCATTGACGTCAATG
GGTGGAGTATTTACGGTAAACTGCCCACTTGGCAGTACATCAAGTGTATC
ATATGCCAAGTACGCCCCCTATTGACGTCAATGACGGTAAATGGCCCGCC
TGGCATTATGCCCAGTACATGACCTTATGGGACTTTCCTACTTGGCAGTAC
ATCTACGTATTAGTCATCGCTATTACTCGAGTGATGCGGTTTTGGCAGTAC
ATCAATGGGCGTGGATAGCGGTTTGACTCACGGGGATTTCCAAGTCTCCA
CCCCATTGACGTCAATGGGAGTTTGTTTTGGCACCAAAATCAACGGGACT
TTCCAAAATGTCGTAACAACTCCGCCCCATTGACGCAAATGGGCGGTAGG

CGTGTACGGTGGGAGGTCTATATAAGCAGAGCTC-3' (SEQ ID NO: 6);

and/or

(2) the functional promoter has the sequence:

5'-TGATGCGGTTTTGGCAGTACATCAATGGGCGTGGATAGCGGTTTGACTCACGGGGATTTCCAAGTCTCCACCCCATTGACGTCAATGGGAGTTTGTTTTGGCACCAAAATCAACGGGACTTTCCAAAATGTCGTAACAACTCCGCCCCATTGACGCAAATGGGCGGTAGGCGTGTACGGTGGGAGGTCTATATAAGCAGAGCTC-3' (SEQ ID NO: 7) ;

and/or

(3) the functional enhancer has the sequence:

5'-TGACATTGATTATTGACTAGTTATTAATAGTAATCAATTACGGGGTCATTAGTTCATAGCCCATATATGGAGTTCCGCGTTACATAACTTACGGTAAATGGCCCGCCTGGCTGACCGCCCAACGACCCCCGCCCATTGACGTCAATAATGACGTATGTTCCCATAGTAACGCCAATAGGGACTTTCCATTGACGTCAATGGGTGGAGTATTTACGGTAAACTGCCCACTTGGCAGTACATCAAGTGTATCATATGCCAAGTACGCCCCCTATTGACGTCAATGACGGTAAATGGCCCGCCTGGCATTATGCCCAGTACATGACCTTATGGGACTTTCCTACTTGGCAGTACATCTACGTATTAGTCATCGCTATTA-3' (SEQ ID NO: 8).

**8.** The nucleic acid molecule of any one of claims 1 to 7, wherein the enhancer of the cytomegalovirus is arranged upstream of the promoter of the cytomegalovirus.

**9.** The nucleic acid molecule of any one of claims 1 to 8, wherein at least one of the one or more internal elements of the CpG island of the aprt gene is arranged upstream of the promoter of the cytomegalovirus or adjacently downstream of the promoter of the cytomegalovirus, wherein optionally (1) at least one of the one or more internal elements of the CpG island of the aprt gene is arranged (i) between the enhancer of the cytomegalovirus and the promoter of the cytomegalovirus, or (ii) adjacently upstream of the enhancer of the cytomegalovirus; and/or (2) at least one of the one or more internal elements of the CpG island of the aprt gene is arranged adjacent to the enhancer of the cytomegalovirus or adjacent to the promoter of the cytomegalovirus.

**10.** The nucleic acid molecule of any one of claims 1 to 9, wherein the nucleic acid molecule is a DNA molecule

**11.** A method of producing a desired polypeptide in cell culture, the method comprising:

- providing a nucleic acid molecule according to claim 1, wherein the nucleic acid molecule further comprises a nucleotide sequence coding for the desired polypeptide, the nucleotide sequence being operably linked to the promoter of the cytomegalovirus and the enhancer of the cytomegalovirus, and

- allowing expression of the desired polypeptide.

**12.** The method of claim 11, wherein (1) the nucleotide sequence coding for the desired polypeptide is a heterologous nucleotide sequence; and/or (2) allowing expression of the desired polypeptide comprises introducing the nucleic acid molecule according to claim 1, optionally comprised in a vector suitable for expression in a host cell, into a suitable host cell.

**13.** A vector comprising the nucleic acid molecule according to any one of claims 1 to 10, wherein optionally (1) the vector is an eukaryotic or a prokaryotic expression vector; and/or (2) the vector is a plasmid.

**14.** A host cell comprising the nucleic acid molecule according to any one of claims 1 to 10.

**15.** Use of the nucleic acid molecule according to any one of claims 1 to 10 for enhancing the expression of a polypeptide of interest in cell culture, wherein the nucleic acid molecule comprises a nucleotide sequence coding for the polypeptide of interest, the nucleotide sequence being operably linked to the promoter of the cytomegalovirus and the enhancer of the cytomegalovirus

**Patentansprüche**

**1.** Ein Nukleinsäuremolekül umfassend einen funktionellen Promoter eines Cytomegaloviruses, einen funktionellen Enhancer eines Cytomegaloviruses und ein oder mehrere interne Elemente der CpG-Insel des aprt (Adeninphosphoribosyltransferase) -Gens, wobei mindestens eines der ein oder mehreren internen Elemente der CpG-Insel des aprt-Gens eine oder mehrere Bindungsstellen für den Transkriptionsfaktor Sp1 umfasst, wobei

(1) das aprt-Gen ein Hamster-aprt-Gen ist und die SP1-Bindungsstelle der CpG-Insel des aprt-Gens die Sequenz: 5'-GCCCCGCCCCGTCCCGCCCC-3' (SEQ ID NO: 1) hat; oder
(2) das aprt-Gen ein Maus-aprt-Gen ist und die SP1-Bindungsstelle der CpG-Insel des aprt-Gens die Sequenz: 5'-CCCGCCC-3' (SEQ ID NO: 2) oder die Sequenz 5'-TCCGCCC-3'(SEQ ID NO: 3) hat.

**2.** Das Nukleinsäuremolekül gemäß Anspruch 1, wobei

(i) wenn das aprt-Gen das Hamster-aprt-Gen ist, das interne Element der CpG-Insel des aprt-Gens die Sequenz:

5′-
TCCAGCAAATGCGTTACTTCCTGCCAAAAGCCAGCCTCCCCGCAACCCA
CTCTCCCAGAGGCCCCGCCCCGTCCCGCCCCCTCCCGGCCTCTCCTCGTG
CTGGATCGCTCCCTAAGGA-3′ (SEQ ID NO: 4)

hat;oder
(ii) wenn das aprt-Gen das Maus-aprt-Gen ist, das interne Element der CpG-Insel des aprt-Gens die Sequenz:

5′-
AGGATGGACATCGCACATCCCCTTTCCACCCATATATCTTTGAGGTAGG
GATGCTTGTGTTTAGGCAGCTCAAGAAATCTAACCCCTGACTCAGGCCC
CACACACACCTCGCAGAGGCCCCGCCTCTCAGCCTGTCCCGCCCCTCGT
GCTAGACCAACCCGCACCCAGAAGCCCCGCCCATCGAGGACGCTCCGC
CCTTGTTCCCCCCGGGATTGACGTG-3′ (SEQ ID NO: 5)

hat.

3. Das Nukleinsäuremolekül gemäß Anspruch 1 oder 2, wobei das eine oder die mehreren internen Elemente der CpG-Insel des aprt-Gens unabhängig in dem Nukleinsäuremolekül in der vorwärts gerichteten Orientierung oder in der rückwärts gerichteten Orientierung, relativ zur Sequenz des Promoters, angeordnet sind.

4. Das Nukleinsäuremolekül gemäß einem der Ansprüche 1 bis 3, wobei das Nukleinsäuremolekül eine Vielzahl der internen Elemente der CpG-Insel des aprt-Gens umfasst.

5. Das Nukleinsäuremolekül gemäß einem der Ansprüche 1 bis 4, wobei das Nukleinsäuremolekül des Weiteren eine expressierbare Nukleotidsequenz umfasst, die für ein Polypeptid von Interesse kodiert, wobei die expressierbare Nukleotidsequenz funktionsfähig an den Promoter des Cytomegaloviruses und den Enhancer des Cytomegaloviruses gebunden ist und vorzugsweise eine heterologe Nukleotidsequenz ist.

6. Das Nukleinsäuremolekül gemäß Anspruch 5, wobei die expressierbare Nukleotidsequenz stromabwärts vom Promoter des Cytomegaloviruses und vom Enhancer des Cytomegaloviruses angeordnet ist.

7. Das Nukleinsäuremolekül gemäß einem der Ansprüche 1 bis 5, wobei

(1) der funktionelle Promoter und der funktionelle Enhancer in der Sequenz:

5′-
TTGACATTGATTATTGACTAGTTATTAATAGTAATCAATTACGGGGTCATTA
GTTCATAGCCCATATATGGAGTTCCGCGTTACATAACTTACGGTAAATGGC
CCGCCTGGCTGACCGCCCAACGACCCCCGCCCATTGACGTCAATAATGACG
TATGTTCCCATAGTAACGCCAATAGGGACTTTCCATTGACGTCAATGGGTG
GAGTATTTACGGTAAACTGCCCACTTGGCAGTACATCAAGTGTATCATATG
CCAAGTACGCCCCCTATTGACGTCAATGACGGTAAATGGCCCGCCTGGCAT
TATGCCCAGTACATGACCTTATGGGACTTTCCTACTTGGCAGTACATCTACG
TATTAGTCATCGCTATTACTCGAGTGATGCGGTTTTGGCAGTACATCAATGG
GCGTGGATAGCGGTTTGACTCACGGGGATTTCCAAGTCTCCACCCCATTGA
CGTCAATGGGAGTTTGTTTTGGCACCAAAATCAACGGGACTTTCCAAAATG
TCGTAACAACTCCGCCCCATTGACGCAAATGGGCGGTAGGCGTGTACGGTG
GGAGGTCTATATAAGCAGAGCTC-3′ (SEQ ID NO: 6)

umfasst sind; und/oder
(2) der funktionelle Promoter die Sequenz:

5′-
TGATGCGGTTTTGGCAGTACATCAATGGGCGTGGATAGCGGTTTGACTCAC
GGGGATTTCCAAGTCTCCACCCCATTGACGTCAATGGGAGTTTGTTTTGGCA
CCAAAATCAACGGGACTTTCCAAAATGTCGTAACAACTCCGCCCCATTGAC
GCAAATGGGCGGTAGGCGTGTACGGTGGGAGGTCTATATAAGCAGAGCTC-
3′ (SEQ ID NO: 7)

hat; und/oder
(3) der funktionelle Enhancer die Sequenz:

5′-
TGACATTGATTATTGACTAGTTATTAATAGTAATCAATTACGGGGTCATTAG
TTCATAGCCCATATATGGAGTTCCGCGTTACATAACTTACGGTAAATGGCC
CGCCTGGCTGACCGCCCAACGACCCCCGCCCATTGACGTCAATAATGACGT
ATGTTCCCATAGTAACGCCAATAGGGACTTTCCATTGACGTCAATGGGTGG
AGTATTTACGGTAAACTGCCCACTTGGCAGTACATCAAGTGTATCATATGC
CAAGTACGCCCCCTATTGACGTCAATGACGGTAAATGGCCCGCCTGGCATT
ATGCCCAGTACATGACCTTATGGGACTTTCCTACTTGGCAGTACATCTACGT
ATTAGTCATCGCTATTA-3′ (SEQ ID NO: 8)

hat.

**8.** Das Nukleinsäuremolekül gemäß einem der Ansprüche 1 bis 7, wobei der Enhancer des Cytomegaloviruses stromaufwärts vom Promoter des Cytomegaloviruses angeordnet ist.

**9.** Das Nukleinsäuremolekül gemäß einem der Ansprüche 1 bis 8, wobei mindestens eines der ein oder mehreren internen Elemente der CpG-Insel des aprt-Gens stromaufwärts vom Promoter des Cytomegaloviruses oder angrenzend stromabwärts vom Promoter des Cytomegaloviruses angeordnet ist, wobei optional (1) mindestens eines des einen oder der mehreren internen Elemente der CpG-Insel des aprt-Gens (i) zwischen dem Enhancer des Cytomegaloviruses und dem Promoter des Cytomegaloviruses oder (ii) angrenzend stromaufwärts vom Enhancer des Cytomegaloviruses angeordnet ist; und/oder (2) mindestens eines der ein oder mehreren internen Elemente der CpG-Insel des aprt-Gens benachbart zu dem Enhancer des Cytomegaloviruses oder benachbart zu dem Promoter des Cytomegaloviruses angeordnet ist.

**10.** Das Nukleinsäuremolekül gemäß einem der Ansprüche 1 bis 9, wobei das Nukleinsäuremolekül ein DNS-Molekül ist.

**11.** Ein Verfahren zur Herstellung eines gewünschten Polypeptids in Zellkultur, wobei das Verfahren umfasst:

- Bereitstellen eines Nukleinsäuremoleküls gemäß Anspruch 1, wobei das Nukleinsäuremolekül des Weiteren eine Nukleotidsequenz umfasst, die für das gewünschte Polypeptid kodiert, wobei die Nukleotidsequenz funktionsfähig an den Promoter des Cytomegaloviruses und den Enhancer des Cytomegaloviruses gebunden ist, und
- Ermöglichen der Expression des gewünschten Polypeptids.

**12.** Das Verfahren gemäß Anspruch 11, wobei (1) die Nukleotidsequenz, die für das gewünschte Polypeptid kodiert, eine heterologe Nukleotidsequenz ist; und/oder (2) das Ermöglichen der Expression des gewünschten Polypeptids das Einbringen des Nukleinsäuremoleküls gemäß Anspruch 1, das optional in einem Vektor enthalten ist, der zur Expression in einer Wirtszelle geeignet ist, in eine geeignete Wirtszelle umfasst.

**13.** Ein Vektor umfassend das Nukleinsäuremolekül gemäß einem der Ansprüche 1 bis 10, wobei optional (1) der Vektor ein eukaryotischer oder ein prokaryotischer Expressionsvektor ist; und/oder (2) der Vektor ein Plasmid ist.

**14.** Eine Wirtszelle umfassend das Nukleinsäuremolekül gemäß einem der Ansprüche 1 bis 10.

**15.** Verwendung eines Nukleinsäuremoleküls gemäß einem der Ansprüche 1 bis 10 zur Verstärkung der Expression eines Polypeptids von Interesse in der Zellkultur, wobei das Nukleinsäuremolekül eine Nukleotidsequenz umfasst, die für das Polypeptid von Interesse kodiert, wobei die Nukeotidsequenz funktionsfähig an den Promoter des Cytomegaloviruses und an den Enhancer des Cytomegaloviruses gebunden ist.

## Revendications

**1.** Molécule d'acide nucléique comprenant un promoteur fonctionnel d'un cytomégalovirus, un amplificateur fonctionnel d'un cytomégalovirus, et un ou plusieurs éléments internes de l'îlot CpG du gène aprt (adénine phosphoribosyltransférase), où au moins l'un des un ou plusieurs éléments internes de l'îlot CpG du gène aprt comprend un ou plusieurs sites de liaison pour le facteur de transcription Sp1, dans laquelle

(1) le gène aprt est un gène aprt de hamster et le site de liaison à SP1 de l'îlot CpG du gène aprt possède la séquence : 5'-GCCCCGCCCCGTCCCGCCCC-3' (SEQ ID NO: 1) ; ou
(2) le gène aprt est le gène aprt murin et le site de liaison à SP1 de l'îlot CpG du gène aprt possède la séquence 5'-CCCGCCC-3' (SEQ ID NO: 2) ou la séquence 5'-TCCGCCC-3' (SEQ ID NO: 3).

**2.** Molécule d'acide nucléique selon la revendication 1, dans laquelle :

(i) lorsque le gène aprt est le gène aprt de hamster, l'élément interne de l'îlot CpG du gène aprt possède la séquence :

5'-
TCCAGCAAATGCGTTACTTCCTGCCAAAAGCCAGCCTCCCCGCAACCCAC
TCTCCCAGAGGCCCCGCCCCGTCCCGCCCCCTCCCGGCCTCTCCTCGTGCT
GGATCGCTCCCTAAGGA-3'
(SEQ ID NO: 4) ;

ou
(ii) lorsque le gène aprt est le gène aprt murin, l'élément interne de l'îlot CpG du gène aprt possède la séquence :

5'-
AGGATGGACATCGCACATCCCCTTTCCACCCATATATCTTTGAGGTAGGG
ATGCTTGTGTTTAGGCAGCTCAAGAAATCTAACCCCTGACTCAGGCCCCA
CACACACCTCGCAGAGGCCCCGCCTCTCAGCCTGTCCCGCCCCTCGTGCTA
GACCAACCCGCACCCAGAAGCCCCGCCCATCGAGGACGCTCCGCCCTTGT
TCCCCCCGGGATTGACGTG-3'
(SEQ ID NO: 5).

**3.** Molécule d'acide nucléique selon la revendication 1 ou 2, dans laquelle le un ou plusieurs éléments internes de l'îlot CpG du gène aprt sont indépendamment arrangés, dans la molécule d'acide nucléique, dans l'orientation sens ou dans l'orientation antisens par rapport à la séquence du promoteur.

**4.** Molécule d'acide nucléique selon l'une quelconque des revendications 1 à 3, où la molécule d'acide nucléique comprend une pluralité de l'élément interne de l'îlot CpG du gène aprt.

**5.** Molécule d'acide nucléique selon l'une quelconque des revendications 1 à 4, où la molécule d'acide nucléique comprend en outre une séquence de nucléotides pouvant être exprimée qui code pour un polypeptide d'intérêt, la séquence de nucléotides pouvant être exprimée étant en liaison fonctionnelle avec le promoteur du cytomégalovirus et l'amplificateur du cytomégalovirus et étant de préférence une séquence de nucléotides hétérologue.

**6.** Molécule d'acide nucléique selon la revendication 5, où la séquence de nucléotides pouvant être exprimée est arrangée en aval du promoteur du cytomégalovirus et de l'amplificateur du cytomégalovirus.

**7.** Molécule d'acide nucléique selon l'une quelconque des revendications 1 à 5, dans laquelle

(1) le promoteur fonctionnel et l'amplificateur fonctionnel sont compris dans la séquence:

5'-
TTGACATTGATTATTGACTAGTTATTAATAGTAATCAATTACGGGGTCATT
AGTTCATAGCCCATATATGGAGTTCCGCGTTACATAACTTACGGTAAATG
GCCCGCCTGGCTGACCGCCCAACGACCCCCGCCCATTGACGTCAATAATG
ACGTATGTTCCCATAGTAACGCCAATAGGGACTTTCCATTGACGTCAATG
GGTGGAGTATTTACGGTAAACTGCCCACTTGGCAGTACATCAAGTGTATC
ATATGCCAAGTACGCCCCCTATTGACGTCAATGACGGTAAATGGCCCGCC
TGGCATTATGCCCAGTACATGACCTTATGGGACTTTCCTACTTGGCAGTAC
ATCTACGTATTAGTCATCGCTATTACTCGAGTGATGCGGTTTTGGCAGTAC
ATCAATGGGCGTGGATAGCGGTTTGACTCACGGGGATTTCCAAGTCTCCA
CCCCATTGACGTCAATGGGAGTTTGTTTTGGCACCAAAATCAACGGGACT
TTCCAAAATGTCGTAACAACTCCGCCCCATTGACGCAAATGGGCGGTAGG
CGTGTACGGTGGGAGGTCTATATAAGCAGAGCTC-3' (SEQ ID NO: 6) ;

et/ou

(2) le promoteur fonctionnel possède la séquence :

5'-
TGATGCGGTTTTGGCAGTACATCAATGGGCGTGGATAGCGGTTTGACTCA
CGGGGATTTCCAAGTCTCCACCCCATTGACGTCAATGGGAGTTTGTTTTGG
CACCAAAATCAACGGGACTTTCCAAAATGTCGTAACAACTCCGCCCCATT
GACGCAAATGGGCGGTAGGCGTGTACGGTGGGAGGTCTATATAAGCAGA
GCTC-3' (SEQ ID NO: 7) ;

et/ou
(3) l'amplificateur fonctionnel possède la séquence :

5'-
TGACATTGATTATTGACTAGTTATTAATAGTAATCAATTACGGGGTCATTA
GTTCATAGCCCATATATGGAGTTCCGCGTTACATAACTTACGGTAAATGGC
CCGCCTGGCTGACCGCCCAACGACCCCCGCCCATTGACGTCAATAATGAC
GTATGTTCCCATAGTAACGCCAATAGGGACTTTCCATTGACGTCAATGGGT
GGAGTATTTACGGTAAACTGCCCACTTGGCAGTACATCAAGTGTATCATA
TGCCAAGTACGCCCCCTATTGACGTCAATGACGGTAAATGGCCCGCCTGG
CATTATGCCCAGTACATGACCTTATGGGACTTTCCTACTTGGCAGTACATC
TACGTATTAGTCATCGCTATTA-3'(SEQ ID NO: 8).

**8.** Molécule d'acide nucléique selon l'une quelconque des revendications 1 à 7, dans laquelle l'amplificateur du cytomégalovirus est arrangé en amont du promoteur du cytomégalovirus.

**9.** Molécule d'acide nucléique selon l'une quelconque des revendications 1 à 8, dans laquelle au moins l'un du un ou plusieurs éléments internes de l'îlot CpG du gène aprt est arrangé en amont du promoteur du cytomégalovirus ou en position adjacente et en aval du promoteur du cytomégalovirus, où facultativement (1) au moins l'un du un ou plusieurs éléments internes de l'îlot CpG du gène aprt est arrangé (i) entre l'amplificateur du cytomégalovirus et le promoteur du cytomégalovirus, ou (ii) en position adjacente et en amont de l'amplificateur du cytomégalovirus ; et/ou (2) au moins l'un du un ou plusieurs éléments internes de l'îlot CpG du gène aprt est arrangé en position adjacente à l'amplificateur du cytomégalovirus ou en position adjacente au promoteur du cytomégalovirus.

**10.** Molécule d'acide nucléique selon l'une quelconque des revendications 1 à 9, où la molécule d'acide nucléique est une molécule d'ADN.

**11.** Procédé de production d'un polypeptide souhaité dans une culture cellulaire, le procédé comprenant :

- la mise à disposition d'une molécule d'acide nucléique selon la revendication 1, où la molécule d'acide nucléique comprend en outre une séquence de nucléotides codant pour le polypeptide souhaité, la séquence de nucléotides étant en liaison fonctionnelle avec le promoteur du cytomégalovirus et l'amplificateur du cytomégalovirus, et
- le fait de permettre l'expression du polypeptide souhaité.

**12.** Procédé selon la revendication 11, dans lequel (1) la séquence de nucléotides codant pour le polypeptide souhaité est une séquence de nucléotides hétérologue ; et/ou (2) le fait de permettre l'expression du polypeptide souhaité comprend l'introduction de la molécule d'acide nucléique selon la revendication 1, facultativement comprise dans un vecteur approprié pour une expression dans une cellule hôte, dans une cellule hôte appropriée.

13. Vecteur comprenant la molécule d'acide nucléique selon l'une quelconque des revendications 1 à 10, où facultativement (1) le vecteur est un vecteur d'expression eucaryote ou procaryote ; et/ou (2) le vecteur est un plasmide.

14. Cellule hôte comprenant la molécule d'acide nucléique selon l'une quelconque des revendications 1 à 10.

15. Utilisation de la molécule d'acide nucléique selon l'une quelconque des revendications 1 à 10 pour améliorer l'expression d'un polypeptide d'intérêt dans une culture cellulaire, où la molécule d'acide nucléique comprend une séquence de nucléotides codant pour le polypeptide d'intérêt, la séquence de nucléotides étant en liaison fonctionnelle avec le promoteur du cytomégalovirus et l'amplificateur du cytomégalovirus.

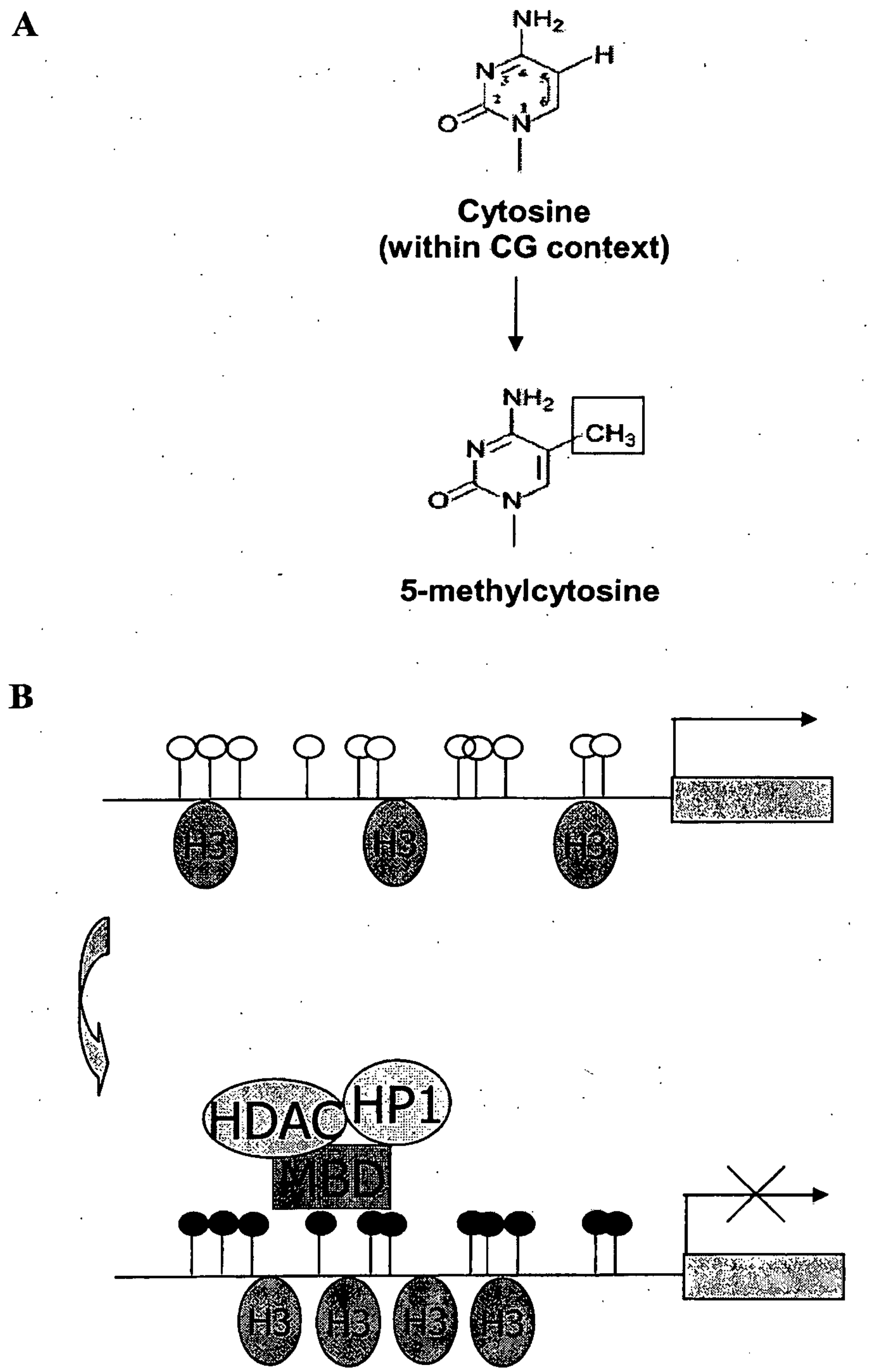
Cytosine
(within CG context)
5-methylcytosine


Figure 1

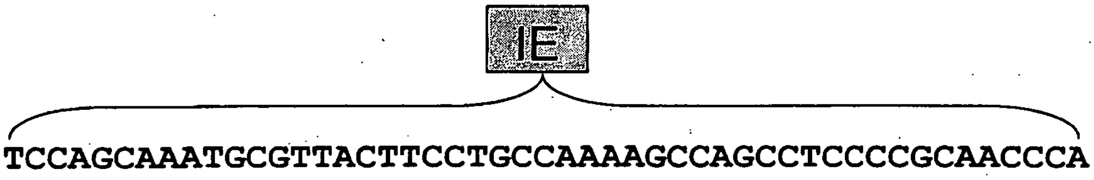


TCCAGCAAATGCGTTACTTCCTGCCAAAAGCCAGCCTCCCCGCAACCCA
CTCTCCCAGAGGCCCCGCCCCGTCCCGCCCCCTCCCGGCCTCTCCTCGT
GCTGGATCGCTCCCTAAGGA

**Figure 2**

**A**

TTGACATTGATTATTGACTAGTTATTAATAGTAATCAATTACGGGGTCATTAGTTCATA
GCCCATATATGGAGTTCCGCGTTACATAACTTACGGTAAATGGCCCGCCTGGCTGACCG
CCCAACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATGG
CCCGCCTGGCATTATGCCCAGTACATGACCTTATGGGACTTTCCTACTTGGCAGTACAT
CTACGTATTAGTCATCGCTATTACTCGAGTGATGCGGTTTTGGCAGTACATCAATGGGC
GTGGATAGCGGTTTGACTCACGGGGATTTCCAAGTCTCCACCCCATTGACGTCAATGGG
AGTTTGTTTTGGCACCAAAATCAACGGGACTTTCCAAAATGTCGTAACAACTCCGCCCC
ATTGACGCAAATGGGCGGTAGGCGTGTACGGTGGGAGGTCTATATAAGCAGAGCTC

**B**

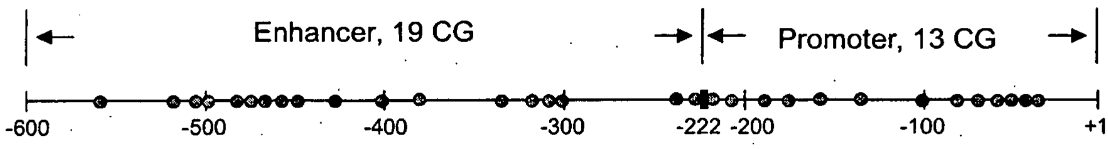


**Figure 3**

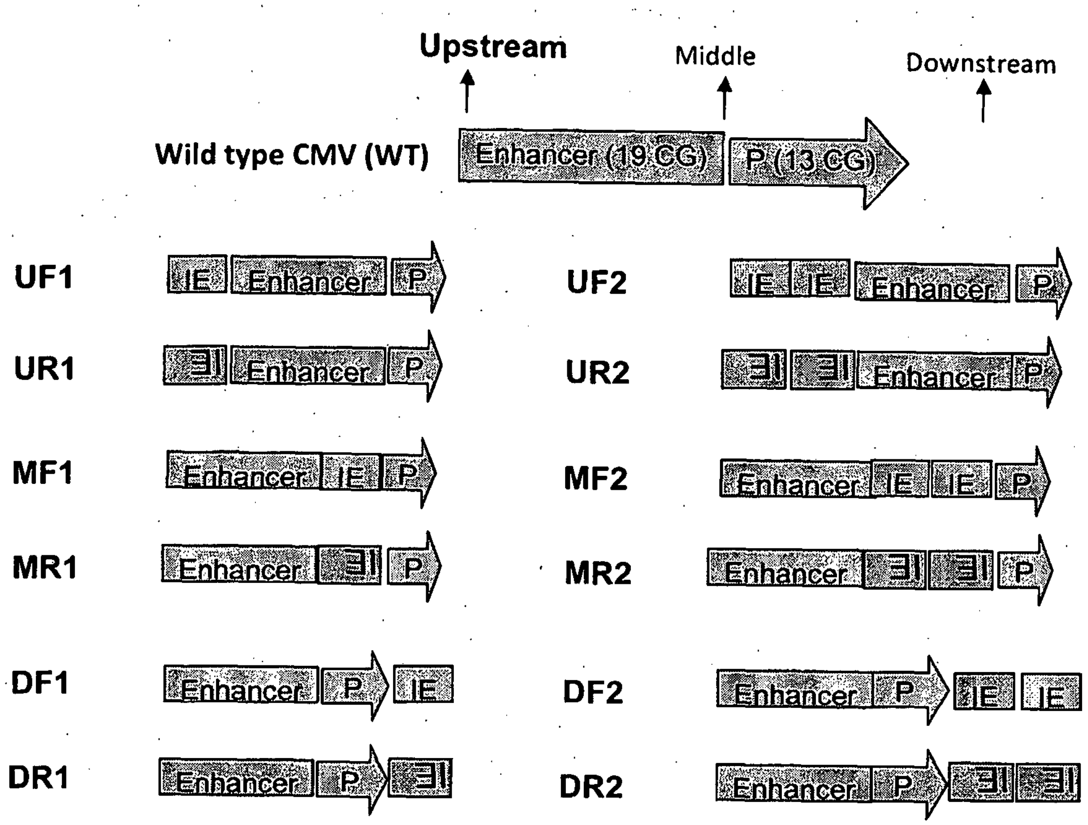
Upstream
Middle
Downstream
Wild type CMV (WT)
Enhancer (19 CG)
P (13 CG)
UF1
IE
Enhancer
P
UF2
IE
IE
Enhancer
P
UR1
Enhancer
P
UR2
Enhancer
P
MF1
Enhancer
IE
P
MF2
Enhancer
IE
IE
P
MR1
Enhancer
P
MR2
Enhancer
P
DF1
Enhancer
P
IE
DF2
Enhancer
P
IE
IE
DR1
Enhancer
P
DR2
Enhancer
P

Figure 4

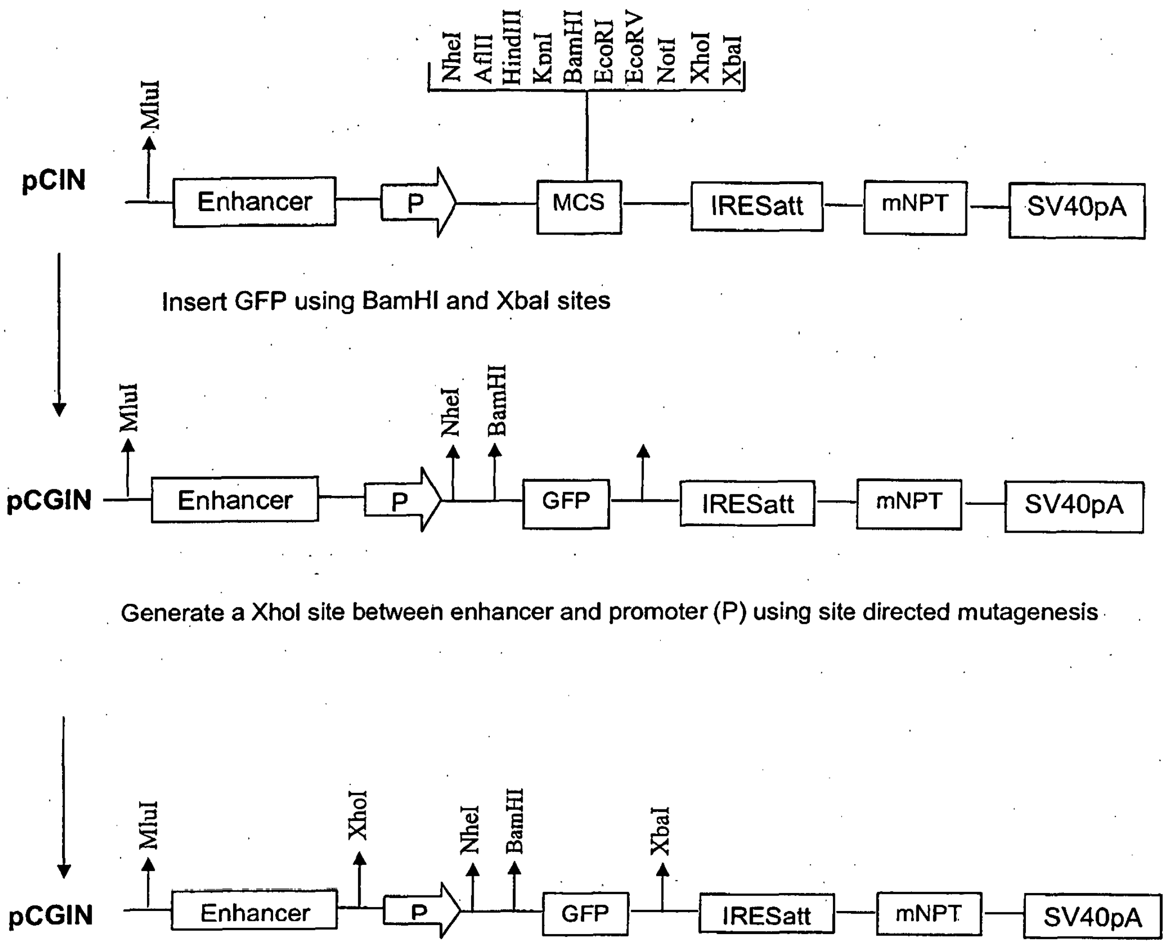
NheI
AflII
HindIII
KpnI
BamHI
EcoRI
EcoRV
NotI
XhoI
XbaI
MluI
pCIN
Enhancer
P
MCS
IRESatt
mNPT
SV40pA
Insert GFP using BamHI and XbaI sites
MluI
NheI
BamHI
pCGIN
Enhancer
P
GFP
IRESatt
mNPT
SV40pA
Generate a XhoI site between enhancer and promoter (P) using site directed mutagenesis
MluI
XhoI
NheI
BamHI
XbaI
pCGIN
Enhancer
P
GFP
IRESatt
mNPT
SV40pA

**Figure 5**

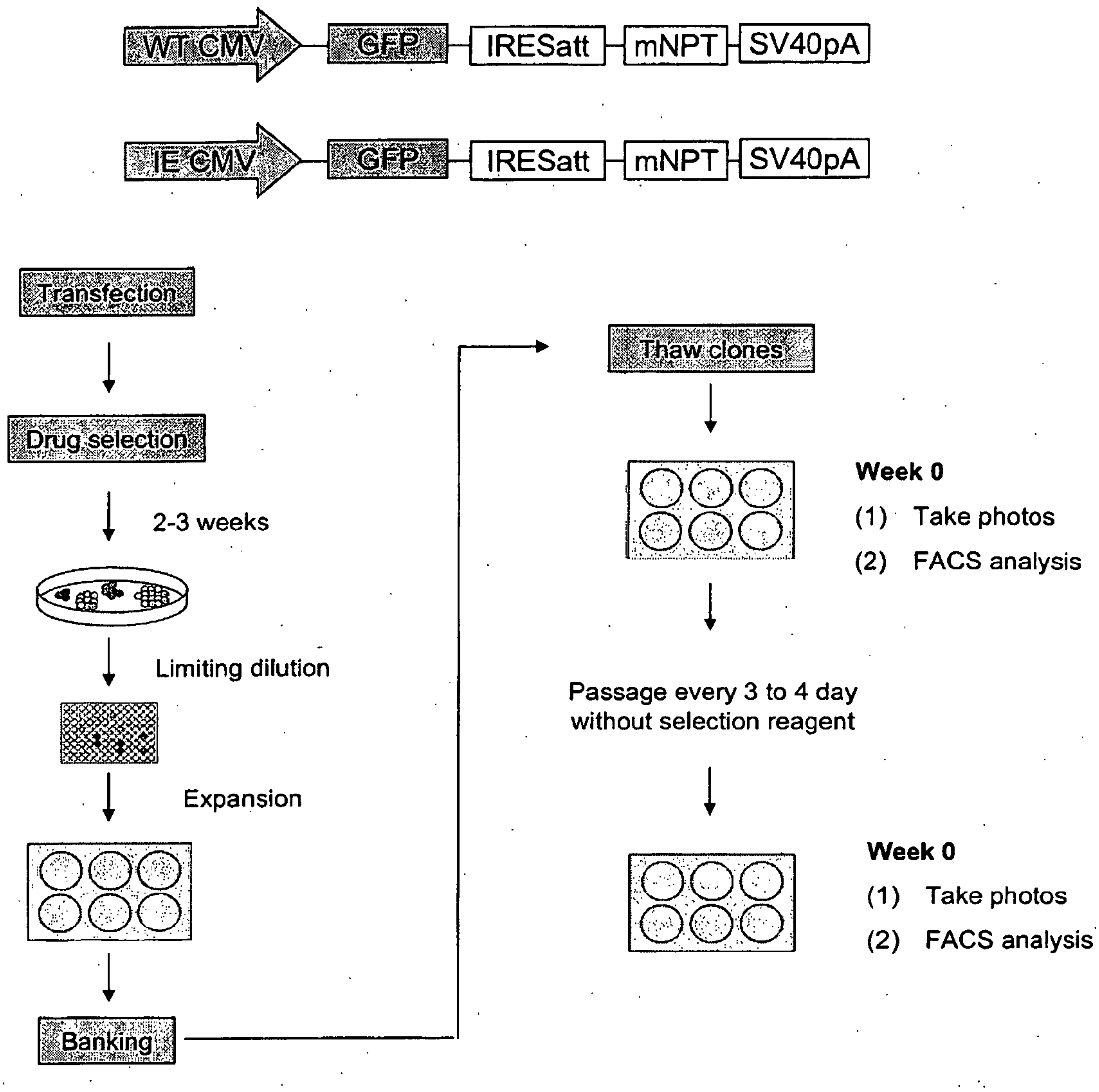
WT CMV
GFP
IRESatt
mNPT
SV40pA
IE CMV
GFP
IRESatt
mNPT
SV40pA
Transfection
Drug selection
2-3 weeks
Limiting dilution
Expansion
Banking
Thaw clones
Week 0
(1) Take photos
(2) FACS analysis
Passage every 3 to 4 day
without selection reagent
Week 0
(1) Take photos
(2) FACS analysis

Figure 6

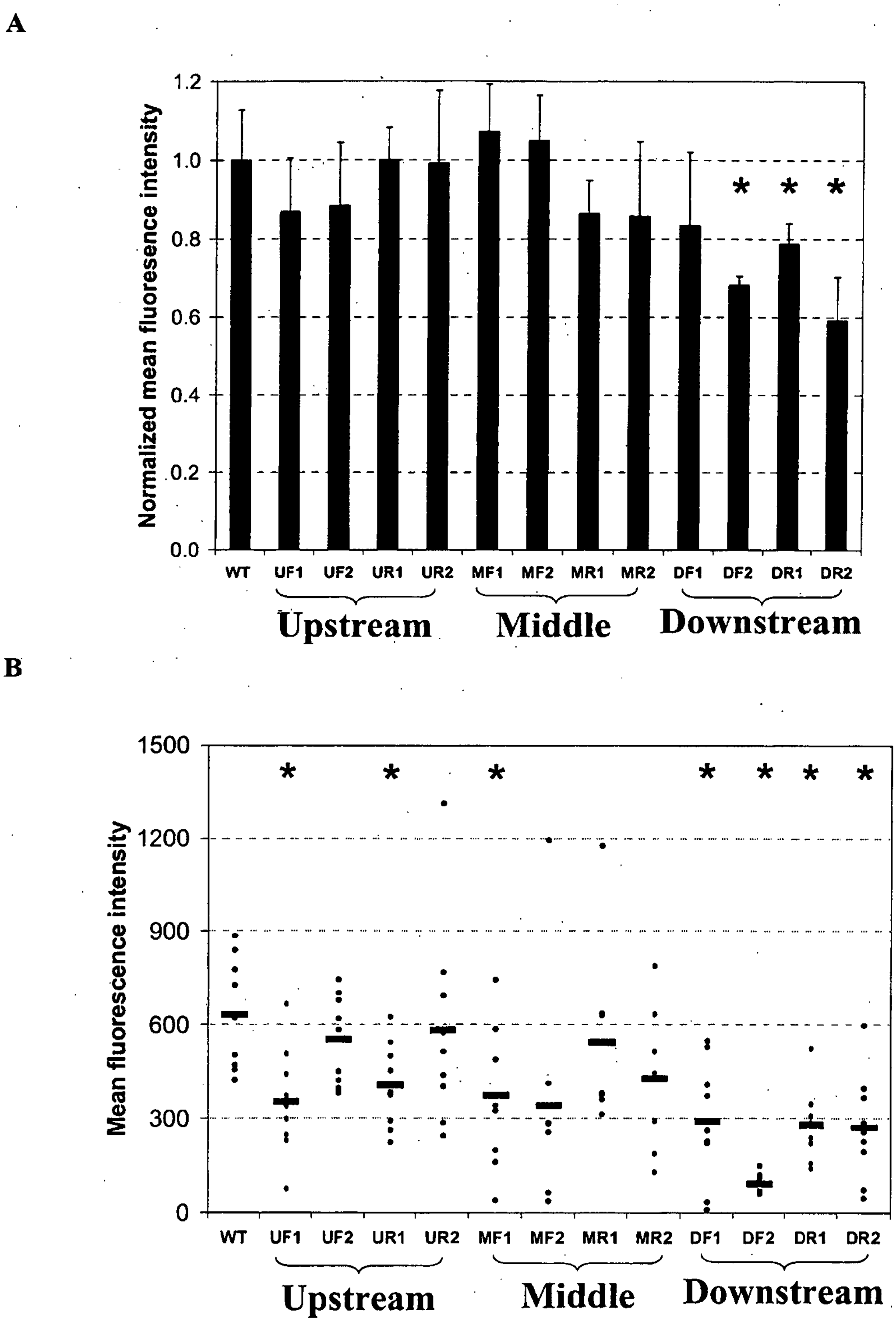
A
Normalized mean fluoresence intensity
1.2
1.0
0.8
0.6
0.4
0.2
0.0
WT UF1 UF2 UR1 UR2 MF1 MF2 MR1 MR2 DF1 DF2 DR1 DR2
Upstream
Middle
Downstream
B
Mean fluorescence intensity
1500
1200
900
600
300
0
WT UF1 UF2 UR1 UR2 MF1 MF2 MR1 MR2 DF1 DF2 DR1 DR2
Upstream
Middle
Downstream

Figure 7

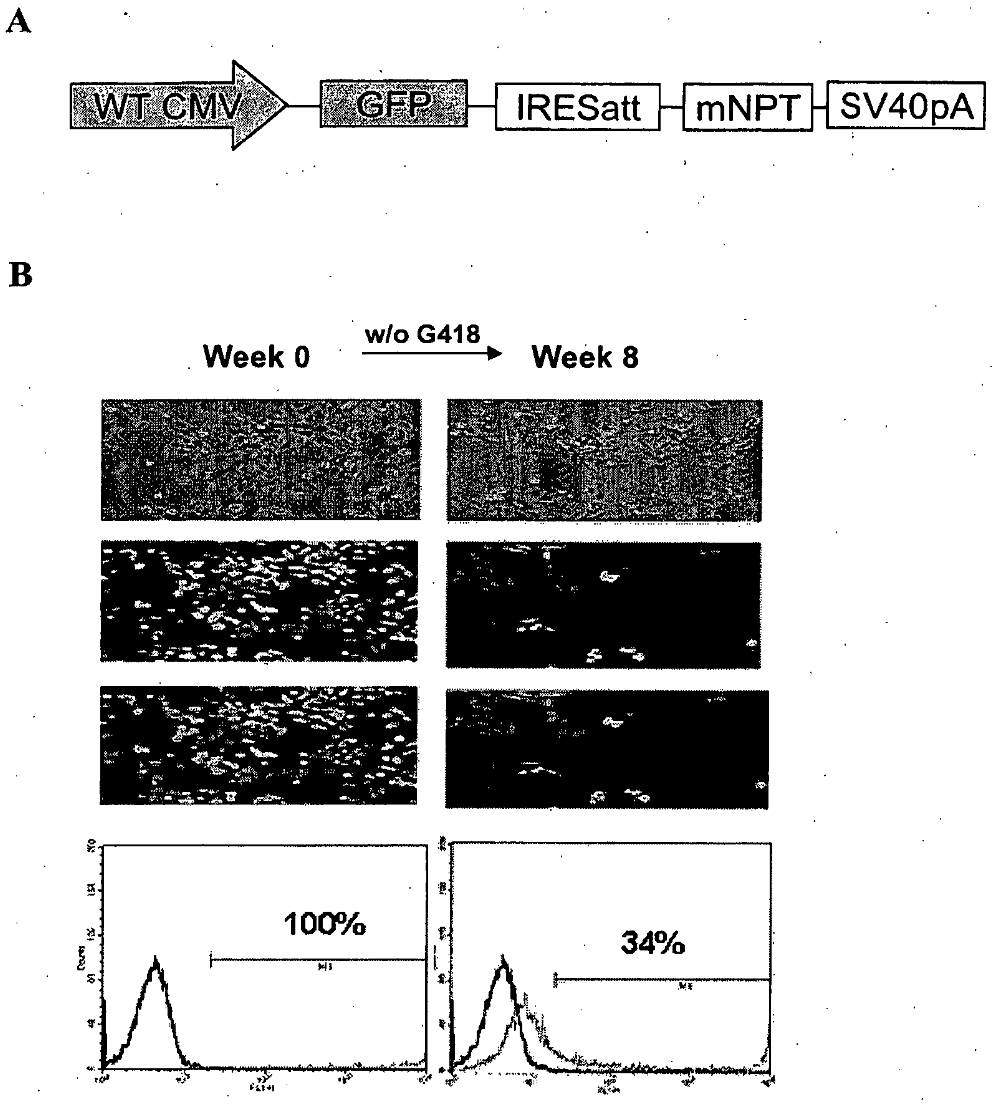


Figure 8

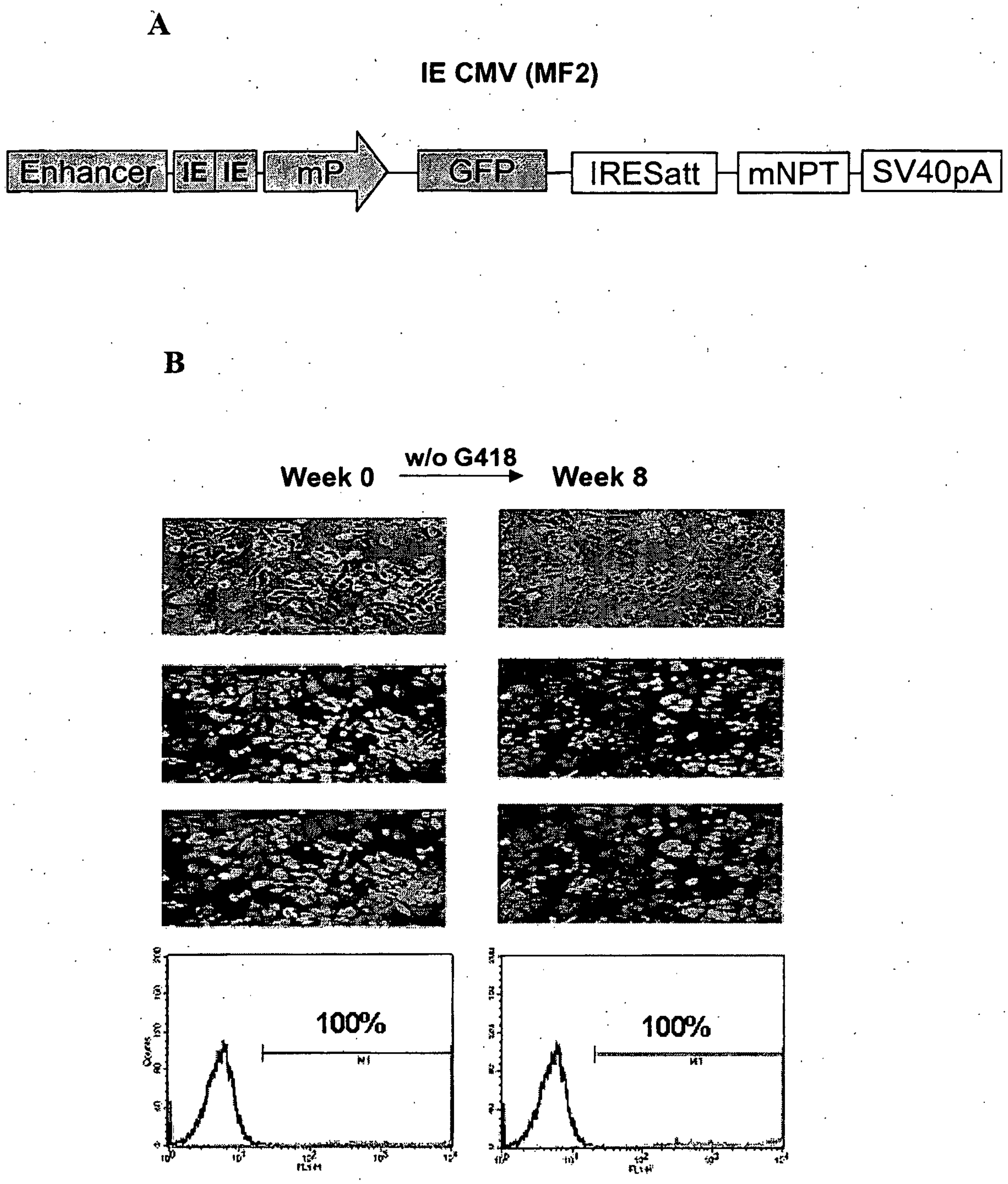
A
IE CMV (MF2)
Enhancer
IE
IE
mP
GFP
IRESatt
mNPT
SV40pA
B
Week 0
w/o G418
Week 8
100%
100%

Figure 9

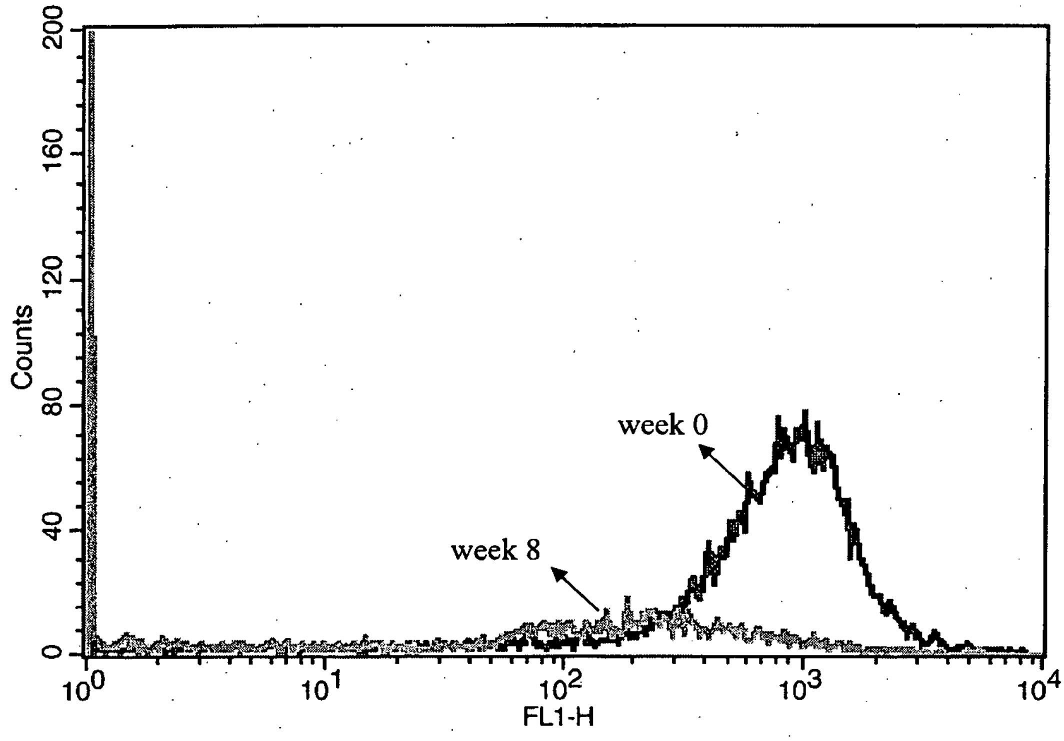


**Figure 10**

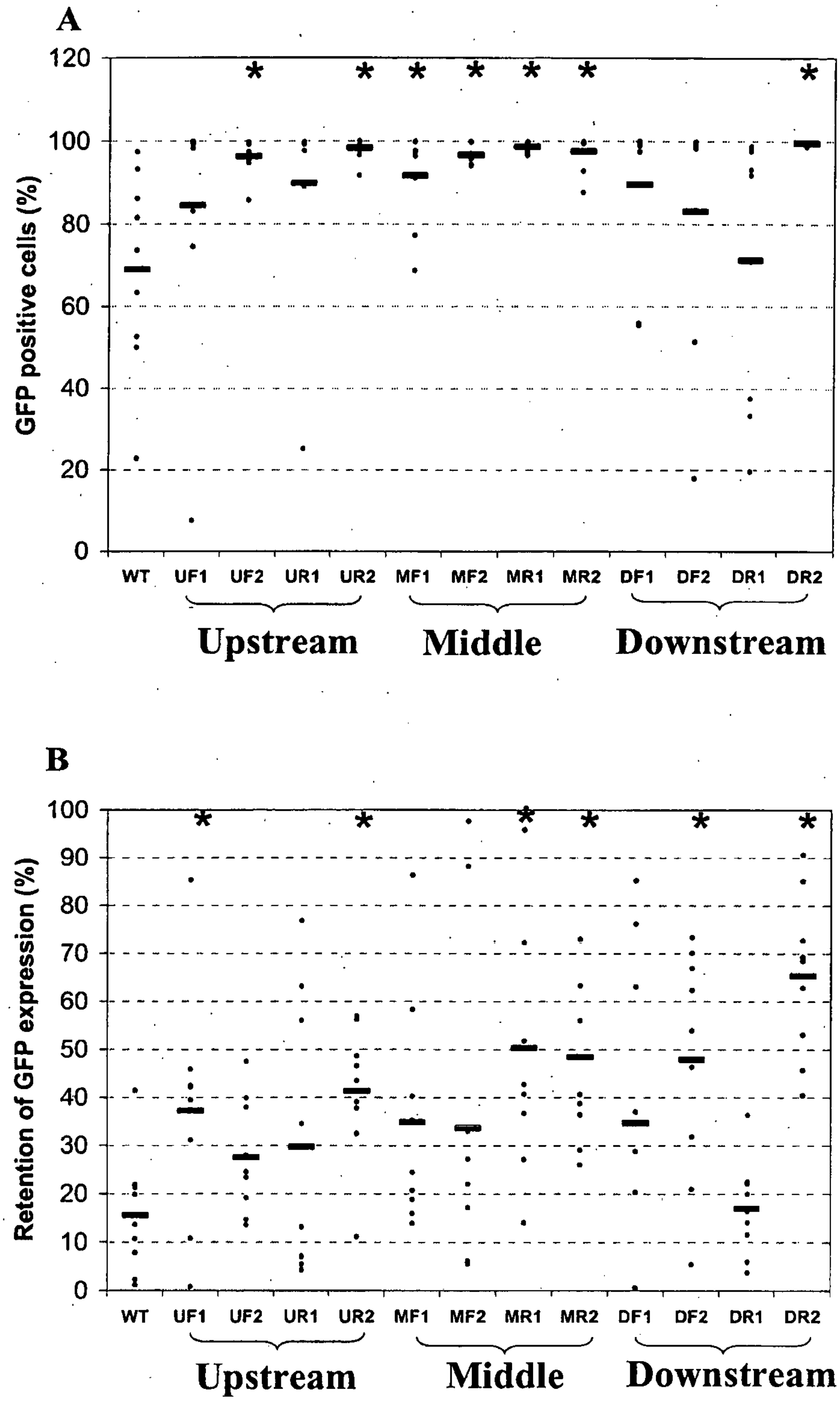
A
GFP positive cells (%)
120
100
80
60
40
20
0
WT UF1 UF2 UR1 UR2 MF1 MF2 MR1 MR2 DF1 DF2 DR1 DR2
Upstream
Middle
Downstream
B
Retention of GFP expression (%)
100
90
80
70
60
50
40
30
20
10
0
WT UF1 UF2 UR1 UR2 MF1 MF2 MR1 MR2 DF1 DF2 DR1 DR2
Upstream
Middle
Downstream

Figure 11

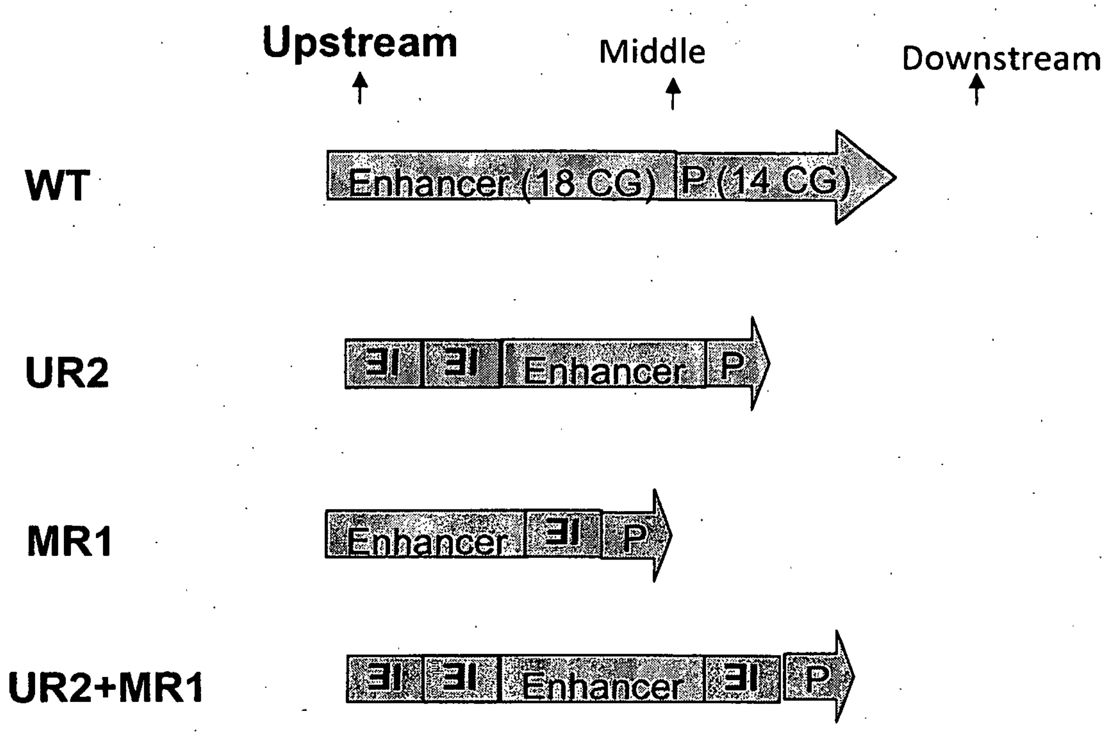
Upstream
Middle
Downstream
WT
Enhancer (18 CG)
P (14 CG)
UR2
Enhancer
P
MR1
Enhancer
P
UR2+MR1
Enhancer
P

Figure 12

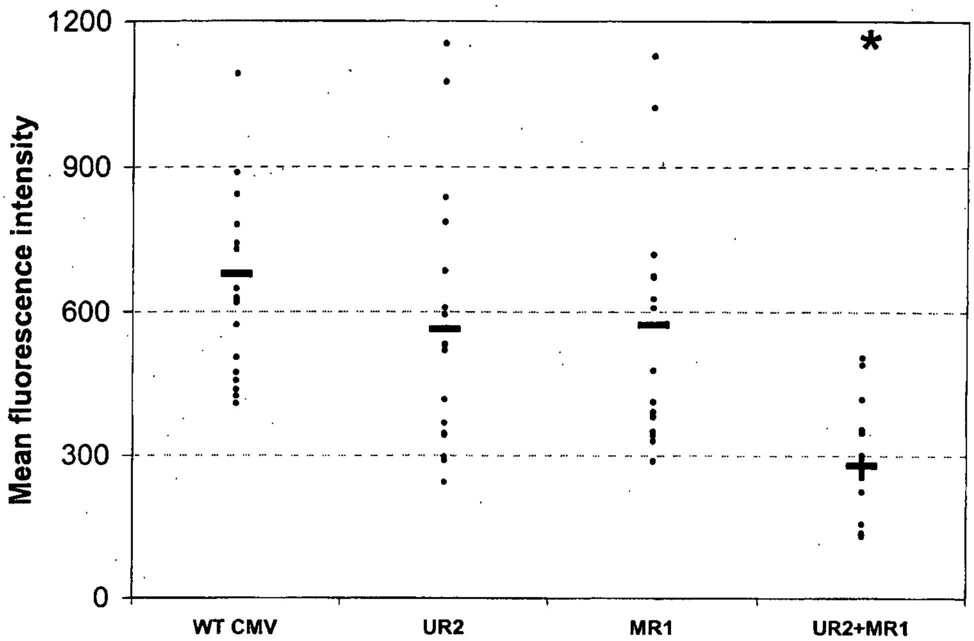


Figure 13

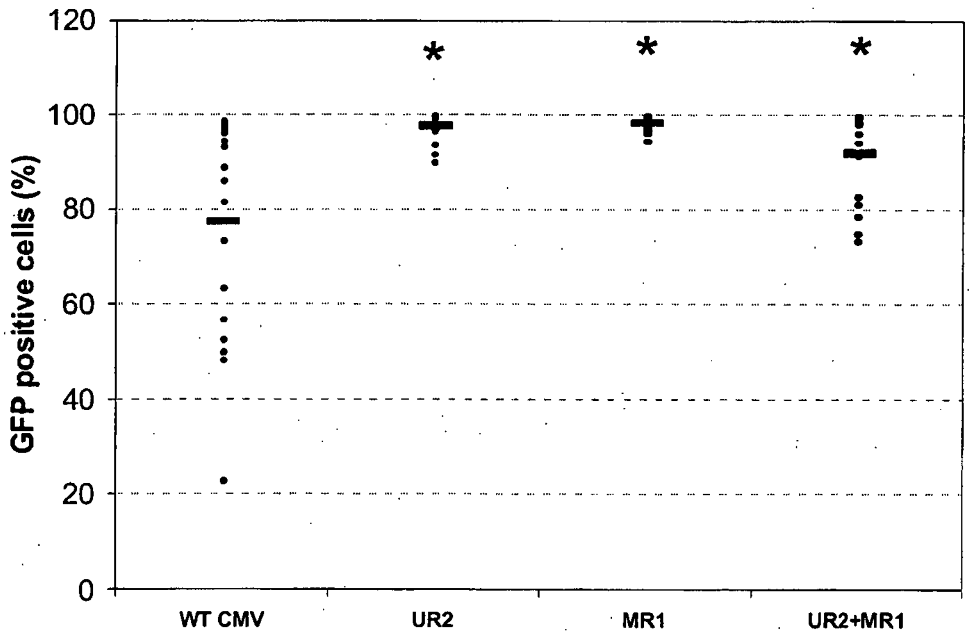


Figure 14

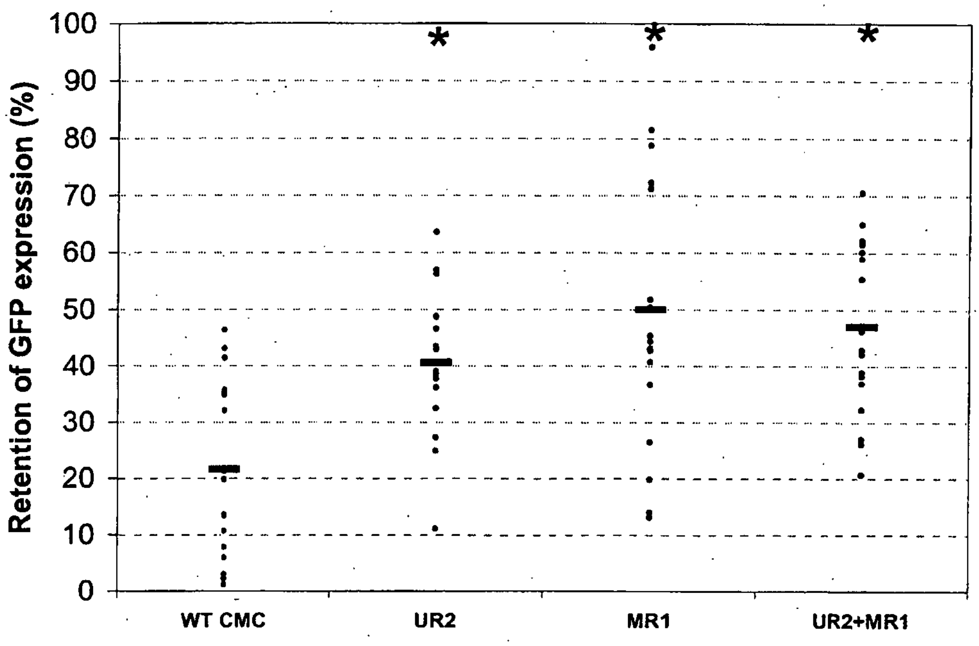
Retention of GFP expression (%)
100
90
80
70
60
50
40
30
20
10
0
*
*
*
WT CMC
UR2
MR1
UR2+MR1

Figure 15

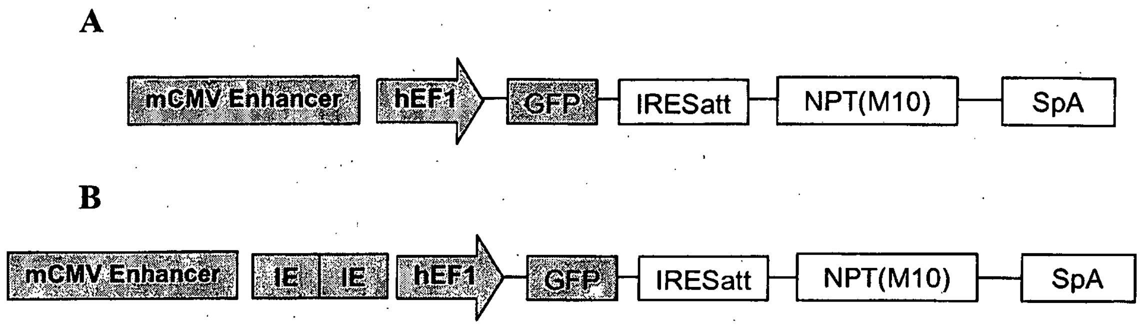


**Figure 16**

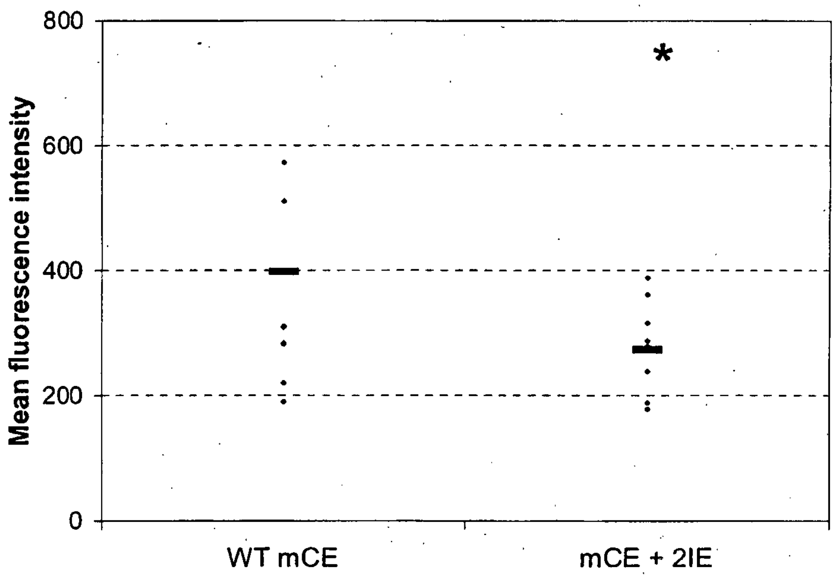


**Figure 17**

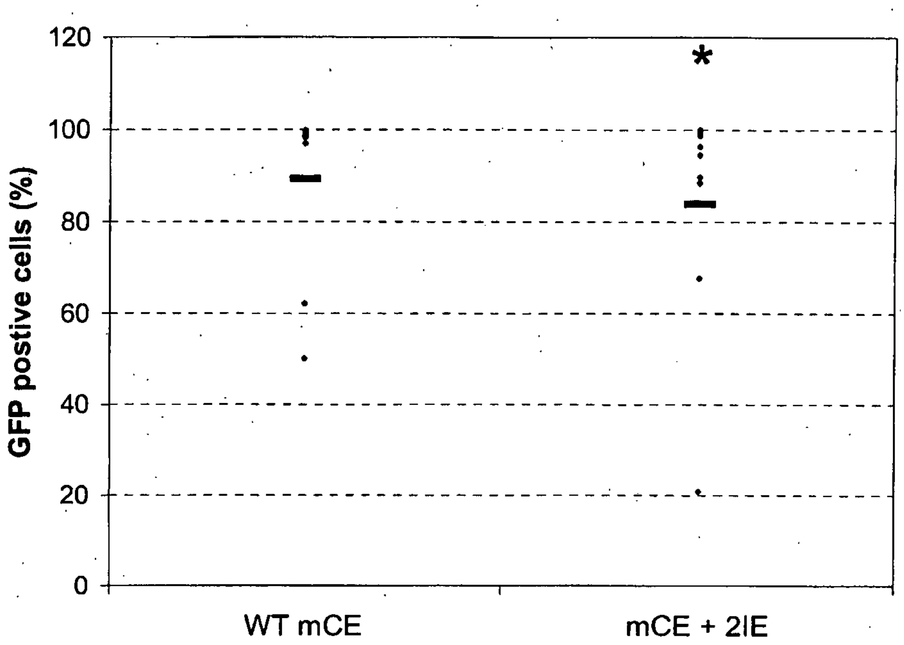


**Figure 18**

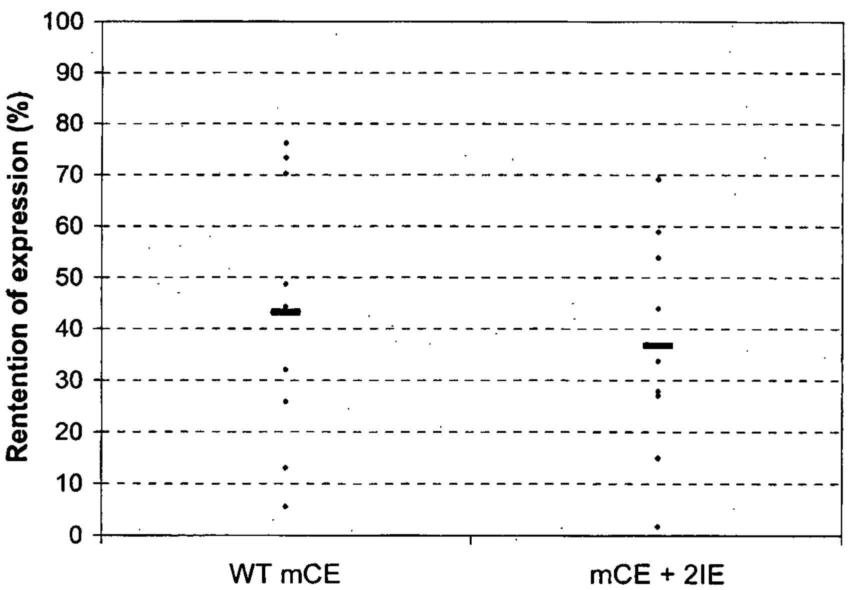


**Figure 19**

A

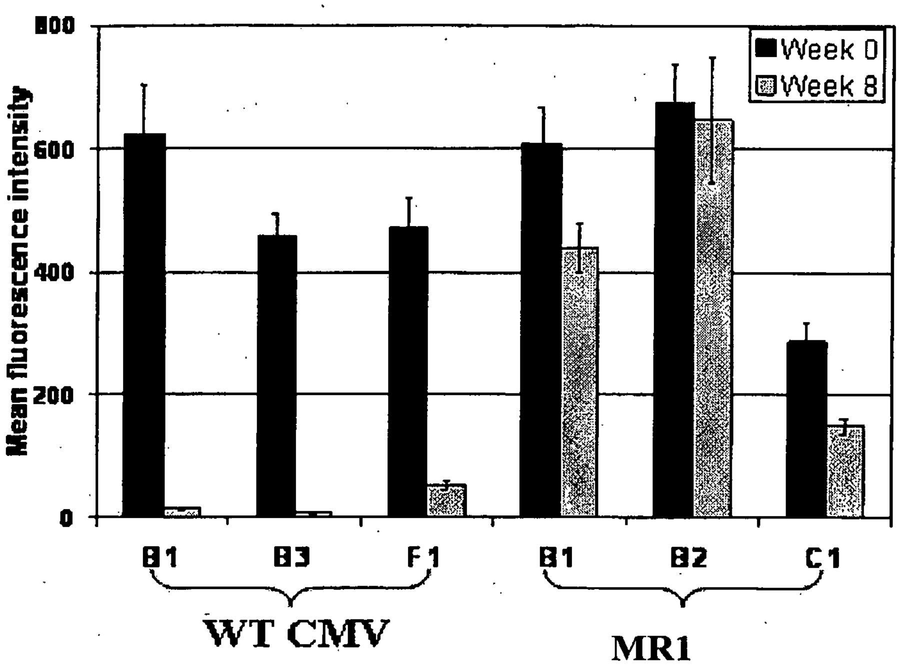
Week 0
Week 8
Mean fluorescence intensity
800
600
400
200
0
B1
B3
F1
B1
B2
C1
WT CMV
MR1

B

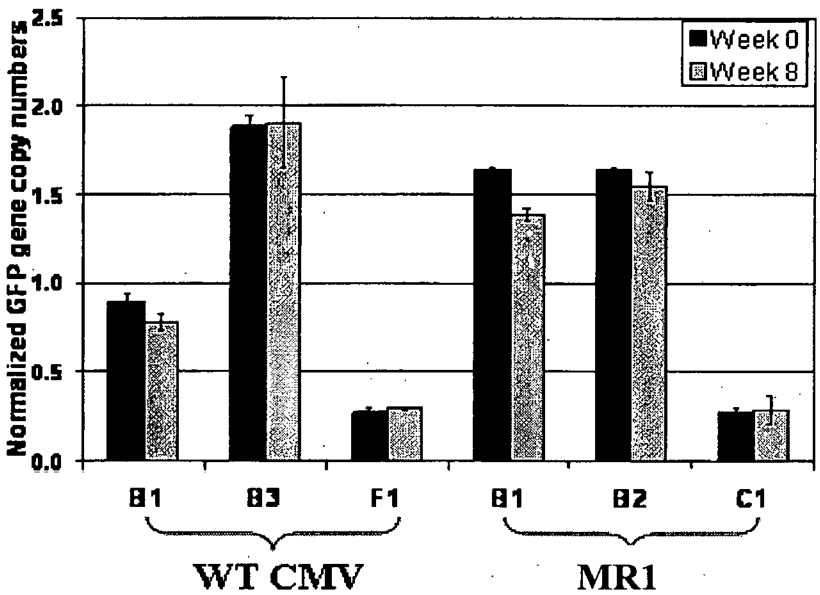
Week 0
Week 8
Normalized GFP gene copy numbers
2.5
2.0
1.5
1.0
0.5
0.0
B1
B3
F1
B1
B2
C1
WT CMV
MR1

Figure 20

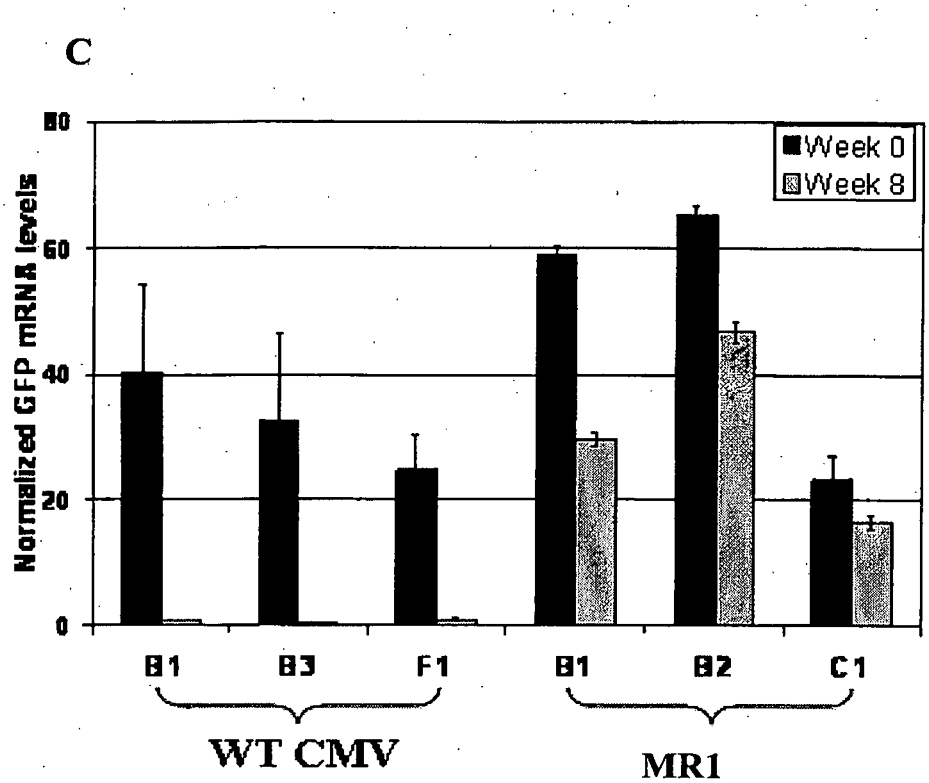
C
Normalized GFP mRNA levels
80
60
40
20
0
Week 0
Week 8
B1
B3
F1
B1
B2
C1
WT CMV
MR1

Figure 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006095156 A
- US 61427121 B


### Non-patent literature cited in the description


- **SENIGL, H ; PLACHY, J. ; HEJNAR, J.** The core element of a CpG island protects avian sarcoma and leukosis virus-derived vectors from transcriptional silencing. *Journal of Virology,* 2008, vol. 82, 7818-7827
- **SIEGFRIED, Z ; EDEN, S.** DNA methylation represses transcription in vivo. *Nature genetics,* 1998, vol. 22, 203-206
- **WILLIAMS S. et al.** *BMC Biotechnology,* 2005, vol. 5, 17
- **BRANDEIS, M. ; FRANK, D. ; KESHET, I.** Elements Protect A Cpg Island From De-Novo Methylation. *Nature,* 1994, vol. 371, 435-438
- **SEED, B.** *Nature,* 1987, vol. 329, 840
- **KAUFINAN et al.** *EMBO J.,* 1987, vol. 6, 187-195
- **LIVAK ; SCHITTGEN.** *Methods,* 2001, vol. 25 (4), 402-408

SEQUENCE LISTING

<110> Agency for Science, Technology and Research

<120> MODIFIED HUMAN CMV PROMOTER THAT IS RESISTANT TO GENE SILENCING

<130> P105962

<150> US 61/427,121
<151> 2010-12-24

<160> 13

<170> PatentIn version 3.5

<210> 1
<211> 20
<212> DNA
<213> Artificial Sequence

<220>
<223> SP1 binding site of CpG island of hamster aprt gene

<400> 1
gccccgcccc gtcccgcccc 20

<210> 2
<211> 7
<212> DNA
<213> Artificial Sequence

<220>
<223> SP1 binding site of CpG island of mouse aprt gene

<400> 2
cccgccc 7

<210> 3
<211> 7
<212> DNA
<213> Artificial Sequence

<220>
<223> SP1 binding site of CpG island of mouse aprt gene

<400> 3
tccgccc 7

<210> 4
<211> 118
<212> DNA
<213> Artificial Sequence

<220>
<223> internal element of CpG island of hamster aprt gene

<400> 4

tccagcaaat gcgttacttc ctgccaaaag ccagcctccc cgcaacccac tctcccagag 60

gccccgcccc gtcccgcccc ctcccggcct ctcctcgtgc tggatcgctc cctaagga 118

<210> 5
<211> 220
<212> DNA
<213> Artificial Sequence

<220>
<223> internal element of CpG island of mouse aprt gene

<400> 5

aggatggaca tcgcacatcc cctttccacc catatatctt tgaggtaggg atgcttgtgt 60

ttaggcagct caagaaatct aacccctgac tcaggcccca cacacacctc gcagaggccc 120

cgcctctcag cctgtcccgc ccctcgtgct agaccaaccc gcacccagaa gccccgccca 180

tcgaggacgc tccgcccttg ttccccccgg gattgacgtg 220

<210> 6
<211> 587
<212> DNA
<213> Human cytomegalovirus

<400> 6

ttgacattga ttattgacta gttattaata gtaatcaatt acggggtcat tagttcatag 60

cccatatatg gagttccgcg ttacataact tacggtaaat ggcccgcctg gctgaccgcc 120

caacgacccc cgcccattga cgtcaataat gacgtatgtt cccatagtaa cgccaatagg 180

gactttccat tgacgtcaat gggtggagta tttacggtaa actgcccact tggcagtaca 240

tcaagtgtat catatgccaa gtacgccccc tattgacgtc aatgacggta aatggcccgc 300

ctggcattat gcccagtaca tgaccttatg ggactttcct acttggcagt acatctacgt 360

attagtcatc gctattactc gagtgatgcg gttttggcag tacatcaatg ggcgtggata 420

gcggtttgac tcacggggat ttccaagtct ccaccccatt gacgtcaatg ggagtttgtt 480

ttggcaccaa aatcaacggg actttccaaa atgtcgtaac aactccgccc cattgacgca 540

aatgggcggt aggcgtgtac ggtgggaggt ctatataagc agagctc 587

<210> 7
<211> 204
<212> DNA
<213> Artificial Sequence

<220>
<223> CMV mini promoter

<400> 7

tgatgcggtt ttggcagtac atcaatgggc gtggatagcg gtttgactca cggggatttc 60

caagtctcca ccccattgac gtcaatggga gtttgttttg gcaccaaaat caacgggact 120

ttccaaaatg tcgtaacaac tccgccccat tgacgcaaat gggcggtagg cgtgtacggt 180

gggaggtcta tataagcaga gctc 204

<210> 8
<211> 376
<212> DNA
<213> Artificial Sequence

<220>
<223> CMV enhancer

<400> 8

tgacattgat tattgactag ttattaatag taatcaatta cggggtcatt agttcatagc 60

ccatatatgg agttccgcgt tacataactt acggtaaatg gcccgcctgg ctgaccgccc 120

aacgaccccc gcccattgac gtcaataatg acgtatgttc ccatagtaac gccaataggg 180

actttccatt gacgtcaatg ggtggagtat ttacggtaaa ctgcccactt ggcagtacat 240

caagtgtatc atatgccaag tacgccccct attgacgtca atgacggtaa atggcccgcc 300

tggcattatg cccagtacat gaccttatgg gactttccta cttggcagta catctacgta 360

ttagtcatcg ctatta 376

<210> 9
<211> 3960
<212> DNA
<213> Cricetulus griseus

<400> 9

ggatccggac aacacccaca ccggcccctc caggtccaga aagctggccc tgcgagaagc 60

gggactgaaa aggcgtgcgg gagccagaaa tccaaaaggg tgccaaggca tgcgtccttt 120

ttccacccag aaataacccc aggctttcaa tttgaggtta tttcaatatc cagcaaatgc 180

gttacttcct gccaaaagcc agcctccccg caacccactc tcccagaggc cccgccccgt 240

cccgccccct cccggcctct cctcgtgctg gatcgctccc taaggacgcc ccgctccaga 300

agccccacct accaaggacg ccccaccctt gttcccggac tggtatgacc ccagcctgct 360

gacatccctc cgccctttct cgtgcacgcg gctatggcgg aatctgagtt gcagctggtg 420

gcgcagcgat ccgcagtttc cccgacttcc ccatccccgg cgtgctgttt aggtgagatc 480

acgagccagc aaggcgttgg agccctgttc ctgggctccc ggcgaggcgc atgggcagtc 540

tcggggatct tgtggggtct ccgcccccct ttccccggcc accagcctct ccttgttccc 600

agggatatct cgcccctcct gaaggacccc gcctccttcc gagcttccat ccgcctcctg 660

gccagtcacc ttaagtccac gcatggcggc aagatcgact acatcgcagg cgagtggcca 720

tgccaggccg tgctggtccc ccactgtgca ggctcccctc ccttccctta tgtcaccctc 780

agtccatccc acacccatcc cttctttctt caacccctaa tcctctttta gcactctgtt 840

tctcttgcct tggtccctcg ttgaccctgg atgagtactg cgtctcccac ccctgctagg 900

tctctgacct ccgccctcag tgcctgttct actagagatg aactctgctc ggtccttgtt 960

cagggccagc ccttctctct tggggtgtga agttggctag cagcctggag gcaggactgt 1020
aagaagcata cgtgtgcttt gagaactttg aggaaggccc tggaacctcc ttgctaggag 1080
tagcacctaa gatgaactag atgctaaaaa atgctgtatc tttggggcac acgagggcat 1140
gcctgggcag gcttagagcc tggtagtctc aggggctgca ccaaagtgta attcttgtgc 1200
taaataactt tcacttacca gtgccaagca cgggcttcag aaacacccta gggtcgctga 1260
atgtccacca ggggagtcag acatgtccag agggtgagaa ccccagagaa ttcggtagcc 1320
ctgacatgtg ctacaattac tgatgcccac ttcctactgg ttcctcctgg ccatacctca 1380
ggaattaggg catgctttct gcctgctaca gtagctcatc ctccctggaa gtgaccccag 1440
acatataccc tgaactgtaa ccgataaagt gcgcctgggc agatgtattt gagaggtggc 1500
aaaagtaaac cataggtgtc cccgagctag atacagaagg cagataacat ccccaaggct 1560
aagctgctgc cccaatagcc atcagccttc tagttatagc tagtaagacc tagtattcct 1620
ggtcaatact attcactcaa tccttacacc tcagccctaa cacgcccccct ctctcatcct 1680
aacaggccta gactccaggg gattcttgtt tggcccctcc ctagctcagg agctgggcct 1740
gggctgtgtg ctcatccgga agcgagggaa gctgccaggc cccacagtgt cagcctccta 1800
tgctctcgag tatggcaagg taagcaggca gtgggtagct gtctaggagt aaatgtgggg 1860
gctcagagag gttaagtcat caggccaggt ttataccacc aggaaacatg gagaagctag 1920
gggtggtggt cacttgttag ctactagact ctcactctac ttcctgtctg caggctgaac 1980
tagaaatcca gaaagacgcc ttagaacctg gccagaaagt ggttgttgta gatgatctcc 2040
tggccactgg aggtaagagc cactctgtag cataaagagg tttcaaaggg ataagcccta 2100
tccggggtgc tgactaagca agagccttac tacctgtgtc tttcctcgtc ccttcacccc 2160
aggaaccatg tgcgctgcct gtgagctgct gggccagcta caggctgagg tggtggagtg 2220
tgtgagcctg gtggagctga cctcacttaa gggcagagag aagctaggat cagtaccatt 2280
cttctctctc ctgcaatatg agtgactgga atggttgctg catccctgcc ctcagcatct 2340
acggttgaac agtggctcag ccttaccaaa gtgacctttg tgagccacca gctgcacttt 2400
ctctaactct attcactcat ttctttggtc agctgatggc catgcctatg gaccacctgg 2460
gtccttgcat tttatatatg tgctgagtcc tggagcagag cagagctact gtgggttatg 2520
acacagcaga tcaataaata gtttggtaca tatggtgctt cctgttgtct tgttgcatgg 2580
atcacgttcc atagctgcca ggtgacagtc cctgtccctt gggattccct gtgcaaatgt 2640
tctccacaga gtcaatccca cacactgccc cggccgtagg gtcttataga accagaatcc 2700
gttttaagca aaaggctttc atcagtagtc tctagagtga catgatggga acaggggctc 2760
agtgtcacct tgcagacctc ttcaaaacag gtggaaacca gtaggcacag ctctgtatag 2820

atttcacagc ctctaggaag ttttctgggc cattccttta ggaccaaaat ggagagagta 2880

gcccaaggga ataccttatt catattttgt tgaacatcaa taccaggttg tttgtaggga 2940

tgctgagcca cacccccagc aagactgttc ccatccagac ttttactggc ctggtcttgc 3000

tagccataga agaaaacatt gcaacaacag cagggcatca tttataggta ttttatggtg 3060

acccattcag tttaatttct gtattacttt tatttaaatc ttacgtgact ttattaaaag 3120

ccaagattaa cagtcacatc caaggtacag aaggttaaat aaaaattttg atctcaaaac 3180

ctatagttat ttccattata tacagaaggc ccaatttcac tcttgccatt cagaactctc 3240

tgtccttatg aaagctggcc agaagcccaa gttctctgtg tgggtcttaa ggctctaagc 3300

aagacagcag ctttcatgag catggagaac aggctaggct ttggcctaag gctcccaact 3360

ggaccttgcc agtacctaga tgagtagttt gtgggacggg catctaacat actctgctct 3420

ccaagtctac ttccccaaca cccacacaag tgctgggtct ggggtaccca aagcctcagg 3480

aaccaattta gcactttatg ggcacaattg ctgcccatac tggcagtaag cccttgggcc 3540

tgtgacactg cgagggatgc tcaagtgcat tgtactgata aagggaacag actatcaggt 3600

aaagaaatga gtcacaaccc ctcagtgtgg atctggtggg tcagcctttc agtgaggcca 3660

gctaagtcag cagctttgtc cagcttgatg taggtgtcca tgcgaatccg atgcaggctc 3720

agccagtctg gcagcaactc agataggagc aacacatgtt tctccatctc ccctgtggaa 3780

gtgcattgtg gttaactgaa gcacagtgcc tggatgactt gcttaactca cttacccagc 3840

ctggtgcctt acacccctca aggcttaacc tgtgtgcagt ggatccaagc agagcctggg 3900

ccctgctctc aaacaccagg gggatgaaca gcaagggcaa gacacatata cgccaagctt 3960

<210> 10
<211> 3066
<212> DNA
<213> Mus musculus

<400> 10

```
gaattcatgc tcacgggctc acaggaaggt ccaagaagga atgtttagaa tccattggac    60
cctccccaca ccctctcctt tgatggagca tgggccaatt tggaggatat cttttgagta   120
attgcaactg cactgaagat gataatggcc attatactca gaggacagtc tttccacacc   180
actacctata gacccaagta ctgtgctggg aaggtagaac cccagttctg tctctggcta   240
tcaggacctt ctggttccac cccaaaacga ggagggcaca ttctgttgca atgcacagga   300
gtgtctgtgg tctcagagaa ggcattcctt acccgccctg ctaccctgct ttcccctgcg   360
ctctagccca cacacagtgc actcccacct ctggacctag actatccatc agctcccttc   420
cggtaatttc aggaaagcag gggctgaatc tcaggccctt gtactatgcg cgagggaagg   480
aacgcaaggc caaaccactc cagcggacct gggcaagacc cgtccctgct cccccaggtc   540
```

cagaagacta gcccctggaa aagcaggact gaaaaagcgt gtgtggggca aaaccaaaaa 600

aggatggaca tcgcacatcc cctttccacc catatatctt tgaggtaggg atgcttgtgt 660

ttaggcagct caagaaatct aacccctgac tcaggcccca cacacacctc gcagaggccc 720

cgcctctcag cctgtcccgc ccctcgtgct agaccaaccc gcacccagaa gccccgccca 780

tcgaggacgc tccgcccttg ttccccccgg gattgacgtg agtttagcgt gctgatacct 840

acctcctccc tgcctcctac acgcacgcgg ccatgtcgga acctgagttg aaactggtgg 900

cgcggcgcat ccgcgtcttc cccgacttcc caatcccggg cgtgctgttc aggtgcggtc 960

acgagccggc gaggcgttgg cgctgtacgc tcatcccccg gcgcaggcgg taggcagcct 1020

cggggatctt gcggggcctc tgcccggcca cacgcgggtc actctcctgt ccttgttcct 1080

agggatatct cgcccctctt gaaagacccg gactccttcc gagcttccat ccgcctcttg 1140

gccagtcacc tgaagtccac gcacagcggc aagatcgact acatcgcagg cgagtggcct 1200

tgctaggtcg tgctcgtccc ccacggtcct agcccctatc ccctttcccc ctcgtgtcac 1260

ccacagtctg ccccacaccc atccattctt cttcgacctc tgacacttcc tccttggttc 1320

ctcactgcct tggacgcttg ttcaccctgg atgaactatg taggagtctc ccttccctgc 1380

taggtaccct aaggcatctg ccctcggtgc ttgttcctag agacgaactc tgctctgtcc 1440

ttgtgtccag aaccaggcct ccctctttta gggcacaaag ctggccagca tcctgacagc 1500

aggctgggag accctggaac ctccagatga cggacatcct tgcttagggg tagcctctgg 1560

gatgaactag atactaaaaa ttaggtaacc ttggttgggc gtggcgtgcc tgggcagacc 1620

tcaagcctgg tagcttcagg ggctgtttct ccccaggact acaccggggc atctttctct 1680

tgttccctca cacaagcttg tgttaaacaa ctgctgtcta cttggctcca tgcctgagct 1740

tgagaaacac cctaggacag ctgaatgtcc accaggagtg tccagaggga gggtgggcac 1800

cccagagaac agagtggcct tggtaagtgc tcggggacca cagactttgc cacttcactt 1860

cctattggta cccttggcca tgctccagaa attagggcat gtatgtatcc ttcccacgac 1920

agctagatgc tgcatttgaa ggtggcaaga ccaccatagg tggccctgag ctgttcagaa 1980

ggcaggtagg atccccaagg ctgagatgat gagttgatgg ctacccagta gccatcaacg 2040

ttcttctaac cgtagtcagc aagacctagt gttcctagca agtgttgacc tcgcccatac 2100

ttggcctcta gattcccatg cccctcagct ccatcccaca accttccctc cttaccctaa 2160

caggtctaga ctccaggggc ttcctgtttg gcccttccct agctcaggag ctgggcgtgg 2220

gctgtgtgct catccggaaa caggggaagc tgccgggccc cactgtgtca gcctcctatt 2280

ctctggagta tgggaaggta agcgagctgt gtgtagagga agggcagggt cttatcacgg 2340

ctaccagtgt ctaggagtaa atgtgggtgc tcagagaggt tgagacattg ggtcaggttt 2400

acaccaccca gaaacgctcg agcctaggga ggtggccact tgttcgcgcc tagactctgt 2460

cttacactac ttcctgtctg caggctgagc tggaaatcca gaaagatgcc ttggaacccg 2520

ggcagagagt ggtcattgtg gatgacctcc tggccacagg aggtaaagaa ccaacccaag 2580

acaaacagac ttcaaagggc cagaccctgt cctgggtgct gactaagcaa agagcttgaa 2640

cacctcctct ttctctgtcc cttcccccca ggaaccatgt ttgcggcctg tgacctgctg 2700

caccagctcc gggctgaagt ggtggagtgt gtgagcctgg tggagctgac ctcgctgaag 2760

ggcagggaga ggctaggacc tataccattc ttctctctcc tccagtatga ctgaggagct 2820

ggctagatgg tcacacccct gctcccagca gcactaggaa ctgcttggtg gctcagccta 2880

ggcgcctaag tgacctttgt gagctaccgg ccgccctttt gtgagtgtta tcactcattc 2940

ctttggtcag ctgatccgcc gtgcctgtgg accccctggat ccttgtactt tgtacacgtc 3000

ccacacaccc tggagcatag cagagctgtg ctactggaga tcaataaacc gttttgatat 3060

gcatgc 3066

<210> 11
<211> 543
<212> DNA
<213> Artificial Sequence

<220>
<223> cDNA nucleotide sequence for the exon regions of mouse aprt gene

<400> 11

atgtcggaac ctgagttgaa actggtggcg cggcgcatcc gcgtcttccc cgacttccca 60

atcccgggcg tgctgttcag ggatatctcg cccctcttga aagacccgga ctccttccga 120

gcttccatcc gcctcttggc cagtcacctg aagtccacgc acagcggcaa gatcgactac 180

atcgcaggtc tagactccag ggcttcctg tttggccctt ccctagctca ggagctgggc 240

gtgggctgtg tgctcatccg gaaacagggg aagctgccgg gccccactgt gtcagcctcc 300

tattctctgg agtatgggaa ggctgagctg gaaatccaga aagatgcctt ggaacccggg 360

cagagagtgg tcattgtgga tgacctcctg gccacaggag gaaccatgtt tgcggcctgt 420

gacctgctgc accagctccg ggctgaagtg gtggagtgtg tgagcctggt ggagctgacc 480

tcgctgaagg gcagggagag gctaggacct ataccattct tctctctcct ccagtatgac 540

tga 543

<210> 12
<211> 70
<212> DNA
<213> Artificial Sequence

<220>
<223> forward primer for internal element

<400> 12

```
tccagcaaat gcgttacttc ctgccaaaag ccagcctccc cgcaacccac tctcccagag    60
gccccgcccc                                                            70
```

<210> 13
<211> 68
<212> DNA
<213> Artificial Sequence

<220>
<223> reverse primer for internal element

<400> 13

```
tccttaggga gcgatccagc acgaggagag gccgggaggg ggcgggacgg ggcggggcct    60
ctgggaga                                                              68
```